# EUROPEAN PATENT APPLICATION

(11) **EP 2 918 166 A1**
(43) Date of publication of application: **16.09.2015**
(21) Application number: 15158148.5
(22) Date of filing: 09.03.2015
(51) Int. Cl.: A01K 67/027

(54) **TTP/MRP14 double knock out mouse model of psoriasis**

(30) Priority: 10.03.2014 EP 14158473
(71) Applicant: Westfälische Wilhelms-Universität Münster, 48149 Münster (DE)
(72) Inventor: Stratis, Athanasios, 48727 Billerbeck (DE); Pap, Thomas, 49549 Ladbergen (DE); Roth, Johannes, 48149 Muenster (DE); Vogl, Thomas, 48149 Muenster (DE)
(74) Representative: Schiweck, Weinzierl & Koch

(57) **Abstract**

The present invention provides a non-human transgenic double knockout animal, in which the following genes are knocked out, gene encoding Tristetraprolin (TTP) and gene encoding myeloid-related protein-14 (MRP14; S100A9). In particular the present invention relates to an animal model for psoriasis, psoriatic arthritis, psoriasis-like diseases and inflammatory diseases of the bowel.

## Description

### FIELD OF THE INVENTION

The present invention generally relates to the field of molecular biology.

More specifically the present invention relates to a non-human transgenic double knockout animal, in which the following genes are knocked out, gene encoding the protein Tristetraprolin (TTP) and gene encoding the protein myeloid-related protein-14 (MRP14; S100A9). In a further aspect, the present invention relates to a tissue, isolated cell(s) or a cell line, characterized in that the cell(s) of said tissue, isolated cell(s) or cell line is/are deficient in the expression of the proteins TTP and MRP14. The present invention further relates to a method for generating said non-human transgenic animal and to methods comprising the non-human transgenic animal or tissue, isolated cell(s) or cell line of the invention.
In particular the non-human transgenic animal, tissue, isolated cell(s) or cell line of the present invention can serve as a model for psoriasis, psoriatic arthritis, psoriasis-like diseases and/or inflammatory diseases of the bowel. Accordingly the present invention also relates to the use of the non-human transgenic animal, tissue, isolated cell(s) or cell line of the invention for identifying and/or testing therapeutic agents for use in the prevention, treatment or alleviation of psoriasis, psoriatic arthritis, psoriasis-like diseases and/or inflammatory diseases of the bowel. Moreover the present invention relates to the use of said non-human transgenic animal, tissue, isolated cell(s) or cell line for identifying and/or characterizing molecular mechanisms underlying psoriasis, psoriatic arthritis, psoriasis-like diseases and/or inflammatory diseases of the bowel.

### BACKGROUND OF THE INVENTION

Psoriasis is a chronic skin disease that affects about 2 % of the population. There are five types of psoriasis: plaque, guttate, inverse, pustular, and erythrodermic. The most common form, plaque psoriasis, is commonly characterized by reddish patches, often covered with silvery scales. In humans psoriasis appears especially on the scalp, trunk, ears and genitalia and the skin over bony prominences, like knees and elbows.

The exact cause of psoriasis is not known. It is assumed that genetic factors are implicated in the epidemiology of psoriasis and that the immune system plays a role in the pathogenesis of psoriasis. Whether a person actually develops psoriasis is hypothesized to depend on particular "trigger factors". Examples of potential trigger factors include systemic infections, injury of the skin (Koebner phenomenon), vaccination, certain medications and various environmental factors such as oxidative stress. Furthermore, since it was shown that patients suffering from psoriasis exhibit an increased intestinal permeability, it is assumed that there is a correlation between psoriasis and the health of the intestine (Humbert P et al., 1991).

Psoriasis not only affects the skin but also other organs. Moreover psoriasis is often associated with other diseases. People suffering from psoriasis are e.g. more likely to develop cardiovascular diseases (e.g. heart disease and stroke), hyperlipidaemia, high blood pressure and diabetes. One of the most important diseases associated with psoriasis is psoriatic arthritis. Around 30 percent of people with chronic psoriasis develop this inflammatory arthritis of the joints. Common symptoms of psoriatic arthritis are pain, swelling and stiffness of joints, painful swelling of fingers and toes (dactylitis), changes to the nails and pain in the area of the sacrum. In addition, psoriatic arthritis is a systemic rheumatic disease that can cause inflammation in body tissues away from the joints and other than the skin, such as the eyes, heart, lungs and kidneys. Furthermore there are signs that there is a correlation between psoriasis and inflammatory diseases of the bowel. Researches show that inflammatory diseases of the bowel aggravate psoriasis (Peslyak MY, 2009). Moreover several studies indicate that psoriasis and inflammatory diseases of the bowel are strictly related diseases, probably sharing common immune-pathogenetic mechanisms. Also the therapeutic overlaps between psoriasis and inflammatory diseases of the bowel support the hypothesis of a common pathogenesis (see e.g. Skroza et al., 2013; Timani et al., 2008).

Inflammatory diseases of the bowel refer to a group of inflammatory disorders of the colon and small intestine. The two most important inflammatory diseases of the bowel are crohn's disease and ulcerative colitis. In addition inflammatory diseases of the bowel include e.g. collagenous colitis, lymphocytic colitis, ischaemic colitis, diversion colitis, behçet's disease and indeterminate colitis. Patients who suffer from an inflammatory disease of the bowel often additionally exhibit diseases that affect multiple other organ systems as well. The skin is one of the most commonly affected organ systems in patients who suffer from an inflammatory disease of the bowel (Huang et al., 2012). Moreover several cutaneous disorders are associated with inflammatory diseases of the bowel. The most frequently associated cutaneous disease is psoriasis which occurs in 7-11 % of the population suffering from an inflammatory disease of the bowel compared to 1-2% of general population (Danese et al., 2005).

The association of inflammatory diseases of the bowel with psoriasis is believed to be both genetically and immunologically related (Georgiou et al., 2006). Furthermore it was shown that there is a striking overlap of loci between diseases of crohn's disease, ulcerative colitis, psoriasis, ankylosing spondylitis and primary sclerosing cholangitis (Cho and Brant, 2011). Some studies suggest that genetic predispositions or HLA linkages for certain specific immune response patterns and common inflammatory pathways may play a role in the association of these diseases (Georgiou et al., 2006; Binus et al., 2011). However, so far the molecular mechanisms causing the relationship of psoriasis and inflammatory disease of the bowel are not yet known.

As used herein psoriasis-like diseases refer to conditions that cause pathological alterations of the body, especially of the skin, which are characteristic for patients suffering from psoriasis. Psoriasis-like diseases include fungal and bacterial infections of the skin, allergic reactions of the skin, intertrigo, lichen planus, lupus erythematosus, skin cancer, eczema (e.g., contact dermatitis, atopic dermatitis, and nummular eczema), parapsoriasis, squamous cell carcinoma, lichen simplex chronicus, pityriasis alba, mycosis fungoides, oncychomycosis, seborrheic dermatitis, tinea and syphilis. Moreover some medication reactions manifest as alterations of the skin similar to psoriasis. Such alterations are encompassed herein in the term psoriasis-like diseases. Due to the similar manifestation of psoriasis and psoriasis-like diseases both may respond to the same therapeutic treatments.

Due to the poorly understood pathophysiology of psoriasis, psoriatic arthritis, inflammatory diseases of the bowel and some of the psoriasis-like diseases, the development of effective treatment modalities for these diseases has proven to be particularly difficult. Moreover these diseases commonly have a chronic recurrent nature, are lifelong conditions and therefore even more challenging to treat.

So far there is no therapy to cure psoriasis and many of established therapeutics, like e.g. glucocorticoids, retinoids or immunosuppressives have side effects which make them unsuitable for a long term use. The same is true for the most inflammatory diseases of the bowel. If no curing surgery of the intestine is possible medical treatments of inflammatory diseases of the bowel are mainly based on immunosuppressive drugs which are often expensive and frequently cause severe side effects.

To develop new therapies for the treatment of psoriasis, psoriatic arthritis, psoriasis-like symptoms and inflammatory diseases of the bowel, preferably therapies with less side effects and lower costs, there is a need to better understand the mechanisms underlying these diseases. Cell models and in particular animal models are extremely valuable tools therefore. A prerequisite for a good model system is that the model reflects the human (and/or animal) pathophysiology. With regard to the state of the art, there are several animal models for psoriasis, e.g. those described in US 6187993, US 5945576, EP 2465346, US 641082, US 7247767, US 2010/0122356, US 2012/0208211. However current psoriasis models just reflect parts of the pathophysiology of psoriasis in humans. So far there is no animal model reflecting all hallmarks of human psoriasis, including skin, arthritic and enteric phenotypes. Moreover there is no animal models that reflect suitably both phenotypes of psoriasis and phenotypes of inflammatory diseases of the bowel and therefore would be suitable for the investigation of the relationship between psoriasis and inflammatory diseases of the bowel.

In sum, there is a need in the art to provide new means and methods that help to diagnose and/or treat patients suffering from psoriasis, psoriatic arthritis, psoriasis-like diseases and/or inflammatory diseases of the bowel. Furthermore there is a need in the art to provide means and methods that help to reveal correlations and commonalities between these diseases, in particular between psoriasis and inflammatory diseases of the bowel. The technical problem underlying the present application is to comply with these needs. This technical problem is solved by providing the embodiments reflected in the claims, described in the description and illustrated in the examples and figures that follow.

### SUMMARY OF THE INVENTION

The present invention overcomes the problems of the prior art by providing, among others, an improved model for psoriasis, psoriatic arthritis, psoriasis-like diseases and/or inflammatory diseases of the bowel. Unlike the prior art the model of the present invention reflects all major histological characteristics of human psoriasis, mimics psoriatic arthritis and exhibits alterations of the intestine characteristic for patients suffering from psoriasis and/or inflammatory diseases of the bowel. The model disclosed herein is therefore i.a. useful for studying the immunopathogenesis of psoriasis, psoriatic arthritis, psoriasis-like diseases and/or inflammatory diseases of the bowel and for evaluating therapeutic agents for ameliorating or preventing said diseases. Moreover the model disclosed herein is useful for studying the correlation between psoriasis and inflammatory diseases of the bowel.

The present invention discloses for the first time that transgenic animals deficient in the expression of the proteins Tristetraprolin (TTP) and myeloid-related protein-14 (MRP14; S100A9) develop severe psoriasis-like phenotypes and can therefore be used as an animal model for psoriasis and diseases associated with psoriasis.

Accordingly, in a first aspect the present invention relates to a non-human transgenic double knockout animal, in which the following two genes are knocked out, gene encoding Tristetraprolin (TTP) and gene encoding myeloid-related protein-14 (Mrp14; S100A9). Said non-human transgenic double knockout animal is referred herein as "non-human transgenic animal of the invention".

In one embodiment the non-human transgenic animal of the invention is further characterized in that the knockout of the gene encoding TTP is a homozygous knockout and the knockout of the gene encoding MRP14 is a homozygous knockout.

In a second embodiment the non-human transgenic animal of the invention is further characterized in that the knockout of the gene encoding TTP is a homozygous knockout and the knockout of the gene encoding MRP14 is a heterozygous knockout.

In another embodiment the non-human transgenic animal of the invention is further characterized in that the knockout of the gene encoding TTP is a heterozygous knockout and the knockout of the gene encoding MRP14 is a homozygous knockout.

In another embodiment the non-human transgenic animal of the invention is further characterized in that the knockout of the gene encoding TTP is a heterozygous knockout and the knockout of the gene encoding MRP14 is a heterozygous knockout.

In particular embodiments the non-human transgenic animal of the invention exhibits at least one phenotype associated with a disease selected from the group consisting of psoriasis, psoriatic arthritis and psoriasis-like diseases. In one embodiment said phenotype is acanthosis. In one embodiment said phenotype is hyperkeratosis. In one embodiment said phenotype is parakeratosis. In one embodiment said phenotype is an increased suprabasal expression of keratin-14 in the epidermis in comparison to a control. In one embodiment said phenotype is a reduced expression of keratin-10 in the stratum spinosum in comparison to a control. In one embodiment said phenotype is a reduced expression of loricrin in the stratum granulosum in comparison to a control. In one embodiment said phenotype is an increased number of macrophages, granulocytes and/or CD3-positive T-cells in the dermis and/or epidermis in comparison to a control. In one embodiment said phenotype is an increased activation of phosphorylated extracellular-signal regulated kinase (pERK) in the epidermis in comparison to a control. In one embodiment said phenotype is an increased amount of interleukin-17, interleukin-22 and/or interleukin-23 in the skin in comparison to a control. In one embodiment said phenotype is an increased expression of myeloid-related protein-8 (MRP8; S100A8) in the epidermis in comparison to a control.

Preferably the non-human transgenic animal of the invention exhibits at least one of the above listed phenotypes associated with psoriasis, psoriatic arthritis and/or psoriasis-like diseases.

In further embodiments the non-human transgenic animal of the invention exhibits at least one phenotype associated with an inflammatory disease of the bowel. Preferably said inflammatory disease of the bowel is selected from the group consisting of Colitis ulcerosa, Crohn's disease, Collagenous colitis, Lymphocytic colitis, Ischaemic colitis, Diversion colitis, Behçet's disease, and Colitis indeterminata. In one embodiment said phenotype is epithelial denudation of the intestine in comparison to a control. In one embodiment said phenotype consists of elongated intestinal crypts, reduction of intestinal crypts and/or destruction of intestinal crypts in comparison to a control. In one embodiment said phenotype is an increased number of ulcers in the intestine in comparison to a control. In one embodiment said phenotype is an increased number of inflammatory infiltrates in the mucosa and/or submucosa of the intestine in comparison to a control. In one embodiment said phenotype is a submucosal edema in the intestine. "Control" preferably refers to is a wild type animal, a TTP single knockout animal and/or a MRP14 single knockout animal.

Preferably the non-human transgenic animal of the invention exhibits at least one of the above listed phenotypes associated with inflammatory diseases of the bowel.

In further embodiments the non-human transgenic animal of the invention exhibits at least one phenotype associated with a disease selected from the group of consisting of psoriasis, psoriatic arthritis, psoriasis-like diseases and inflammatory diseases of the bowel as defined above. In particular embodiments the non-human transgenic animal of the invention develops a macroscopically observable expression of said phenotype postnatally. In other embodiments said phenotype is prevented and/or alleviated by suppressing or inhibiting Tumor necrosis factor alpha (TNF) in the animal of the invention. Preferably TNF is inhibited by subjecting an anti-TNF therapy to the animal of the invention. In one embodiment said anti-TNF therapy comprises administering a TNF neutralizing antibody to the animal of the invention, wherein said TNF neutralizing antibody is optionally Certolizumab.

In preferred embodiments the non-human transgenic animal of the invention is a non-human mammal or a fish. In particular embodiments the non-human transgenic animal of the invention is a mouse, rat, rabbit, guinea pig, or zebrafish. In one embodiment the non-human transgenic animal of the invention is a mouse.

In one embodiment the non-human transgenic animal of the invention is for use in identifying and/or characterizing mechanisms underlying psoriasis, psoriatic arthritis, psoriasis-like diseases and/or inflammatory diseases of the bowel.

In another embodiment the non-human transgenic animal of the invention is for use in identifying and/or testing compounds for use in the prevention, treatment or alleviation of a disease selected from the group consisting of psoriasis, psoriatic arthritis, psoriasis-like diseases and inflammatory diseases of the bowel.

In a second aspect, the present invention relates to a method for generating a non-human transgenic animal of the invention. In one embodiment said method comprises:
(i) crossing a TTP single knockout animal with a MRP14 single knockout animal of the same species; and optionally
(ii) backcrossing of the descendants obtained in step (i) into a genetic background strain.

In another aspect, the present invention relates to a method for identifying and/or testing a therapeutic agent for use in the prevention, treatment or alleviation of psoriasis, psoriatic arthritis, a psoriasis-like disease and/or an inflammatory disease of the bowel of a subject, the method comprising:
(i) administering a test agent to the non-human transgenic animal of the invention; and
(ii) determining at least one phenotype associated with psoriasis, psoriatic arthritis, psoriasis-like diseases and inflammatory diseases of the bowel in a group of said non-human transgenic animals of the invention to which the test agent is administered.

In another aspect, the present invention relates to a therapeutic agent identified by a method comprising the animal of the invention e.g. the method for identifying and/or testing a therapeutic agent defined above. In a preferred embodiment the therapeutic agent of the invention is for use in the prevention, treatment and/or alleviation of at least one disease of a subject, wherein said disease is psoriasis, psoriatic arthritis, a psoriasis-like disease and/or an inflammatory disease of the bowel.

In a further aspect, the present invention relates to a pharmaceutical composition comprising a therapeutic agent identified by a method comprising the animal of the invention e.g. the method for identifying and/or testing a therapeutic agent defined above. In a preferred embodiment the pharmaceutical composition of the invention is for use in the prevention, treatment or alleviation of a disease of a subject, wherein said disease is psoriasis, psoriatic arthritis, a psoriasis-like disease and/or an inflammatory disease of the bowel.

In another aspect, the present invention encompasses a tissue, isolated cell(s) or a cell line, characterized in that the cell(s) of said tissue, isolated cell(s) or cell line are deficient in the expression of the proteins TTP and MRP14. In a further aspect the present invention encompasses a tissue, isolated cell(s) or cell line derived from the non-human transgenic animal of the invention.

In another aspect the present invention relates to the use of the non-human transgenic animal of the invention or the tissue, isolated cell(s) or cell line of the invention for identifying and/or characterizing molecular and cellular mechanisms underlying psoriasis, psoriatic arthritis, psoriasis-like diseases and/or inflammatory diseases of the bowel.

Another aspect of the invention is the use of the non-human transgenic animal of the invention or the tissue, isolated cell(s) or cell line of the invention for identifying and/or testing a therapeutic agent or pharmaceutical composition for use in the prevention, treatment or alleviation of psoriasis, psoriatic arthritis, psoriasis-like diseases and/or inflammatory diseases of the bowel.

### DESCRIPTION OF THE FIGURES

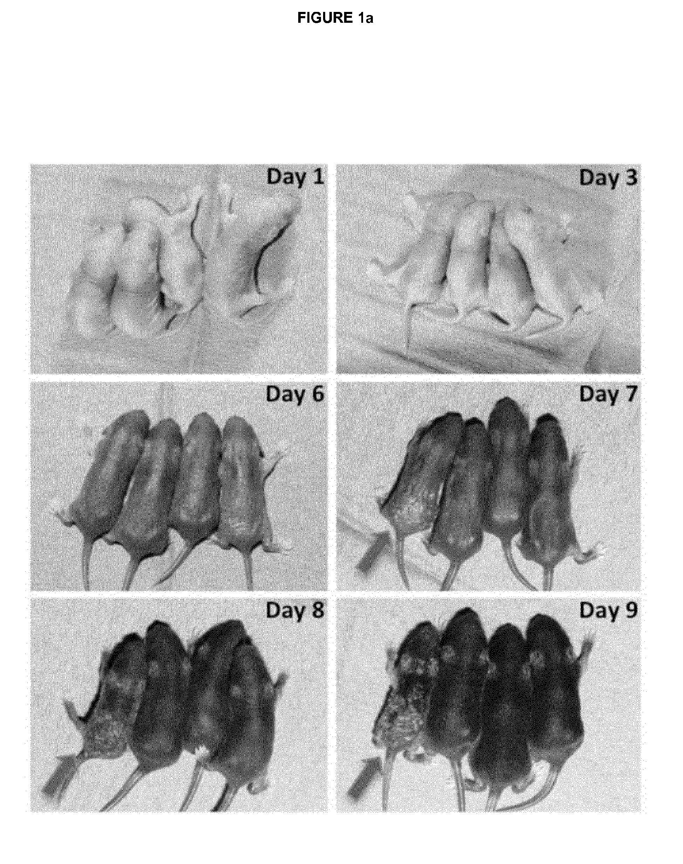

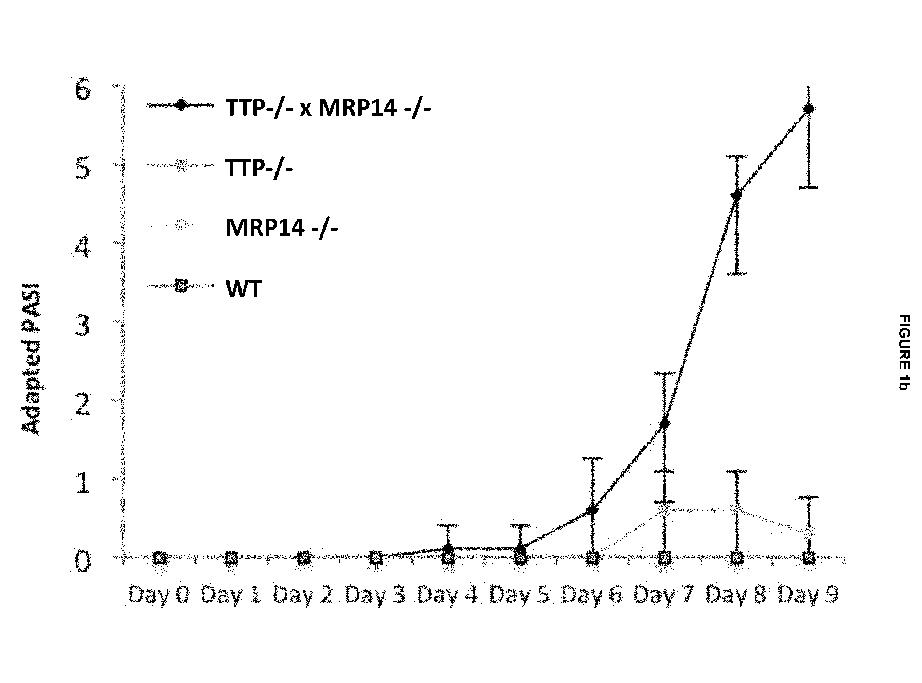

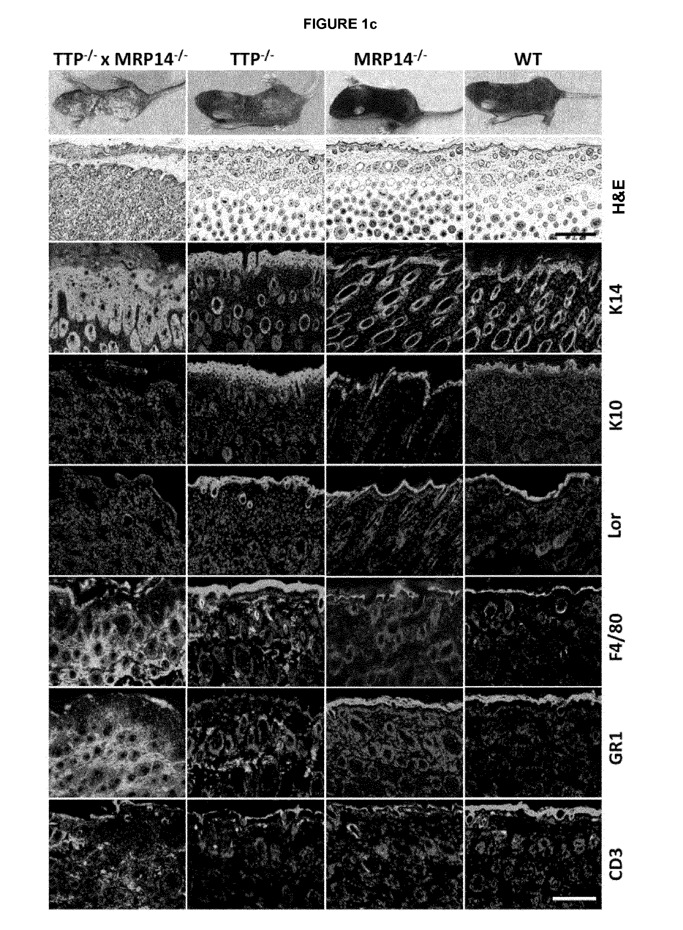

**Figure 1****:** Phenotype of TTP-/- x MRP14-/- mice. **1a,** Disease progression of TTP-/- x MRP14-/- mice at day 1-9. The arrow mark indicates the TTP-/- x MRP14-/- mouse and the macroscopic progression of the psoriatic phenotype. **1 b,** Adapted PASI score of TTP-/- x MRP14-/- mice, TTP-/- mice, MRP14-/- mice and wild type (WT) mice. **1c,** Photographs and skin sections of TTP-/- x MRP14-/-, TTP-/-, MRP14-/- and WT mice at day 9. Sections were stained with hematoxylin/eosin (H&E) or immunostained for the indicated epidermal differentiation markers keratin-14 (K14), keratin-10 (K10) and loricrin (Lor) or invading T cells (CD3), granulocytes (Gr1) and macrophages (F4/80) (green signal). Red counterstaining shows tissue structure. Scale bar: 200 µm (H&E); 50 µm (immunostaining).

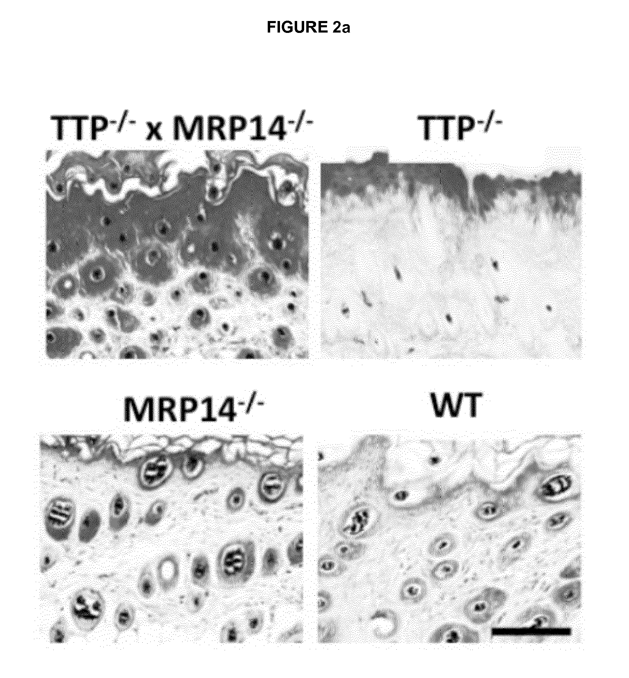

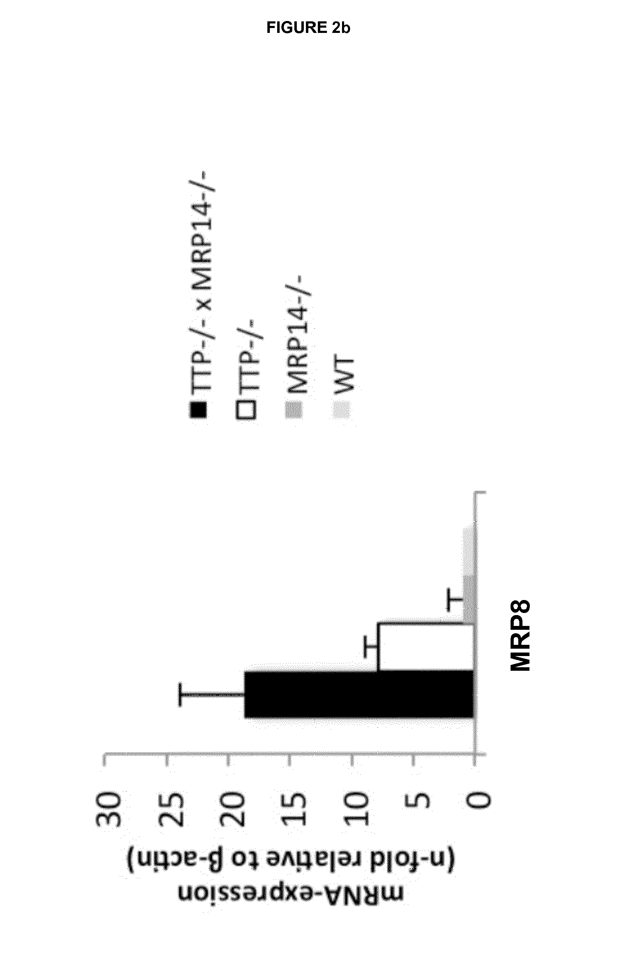

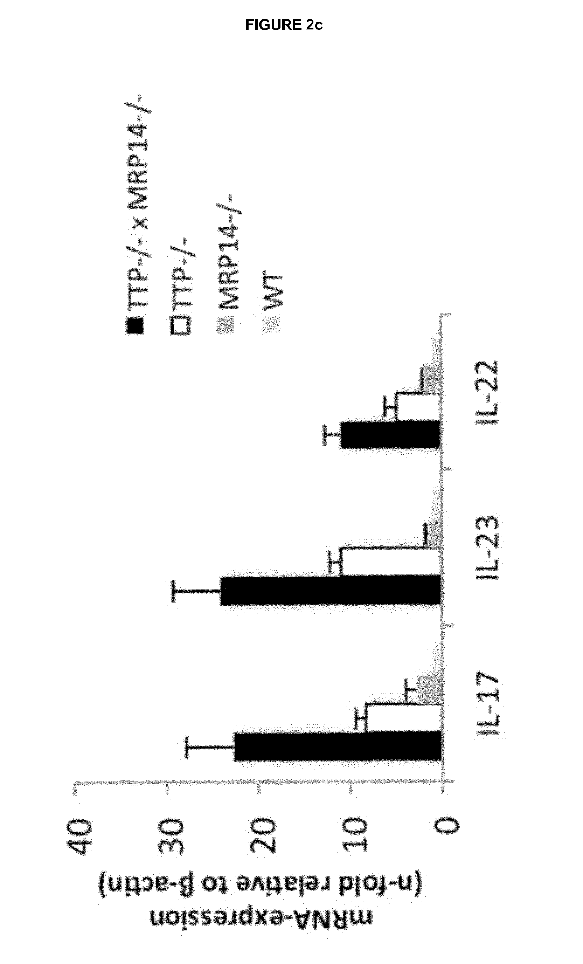

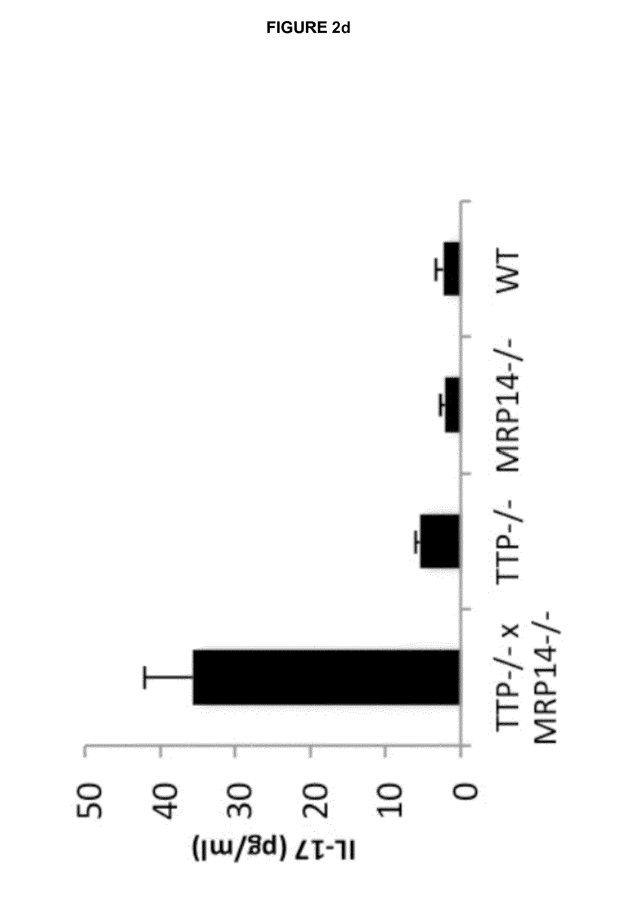

**Figure 2****:** Expression of MRP14, interleuckin-17 (IL-17), interleukin-23 (IL-23) and interleukin-22 (IL-22) in the skin of TTP-/- x MRP14_{-/-} mice at day 9. **2a.** Skin sections were stained with an antibody recognizing MRP8 (red) Scale bar: 50 µm. **2b,** mRNA levels of MRP14 and **2c,** mRNA levels of IL-17, IL-23 and IL-22 in the skin were measured by real-time quantitative PCR. **2d,** IL-17 ELISA of skin samples.

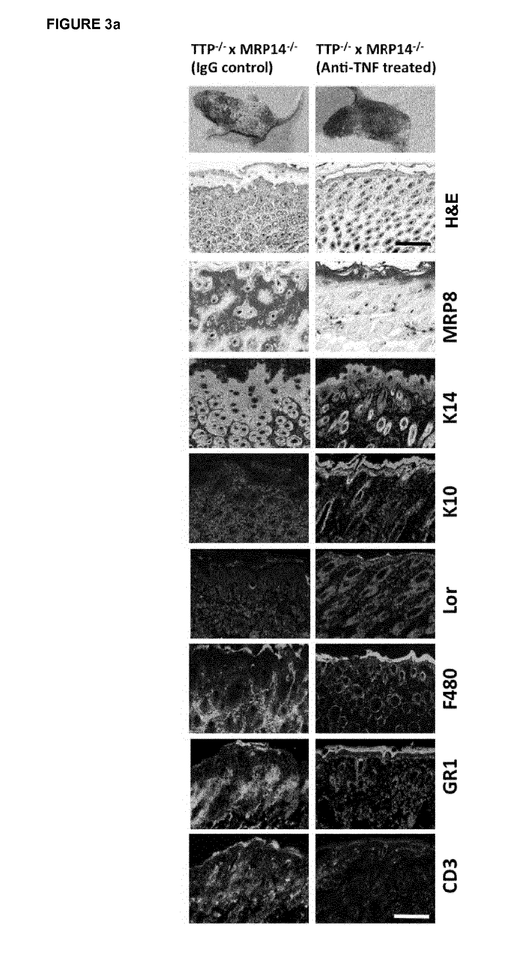

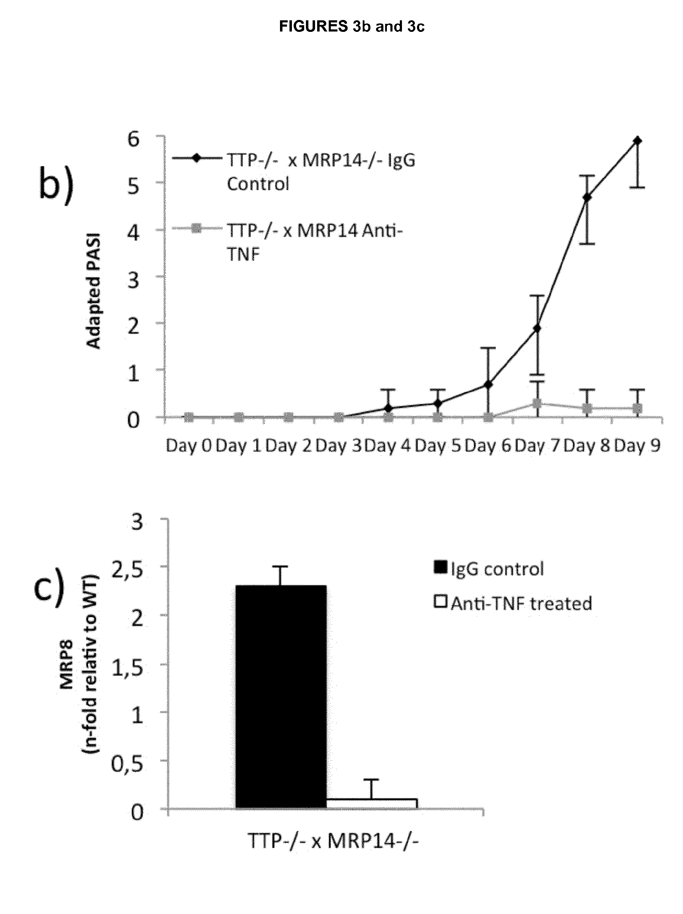

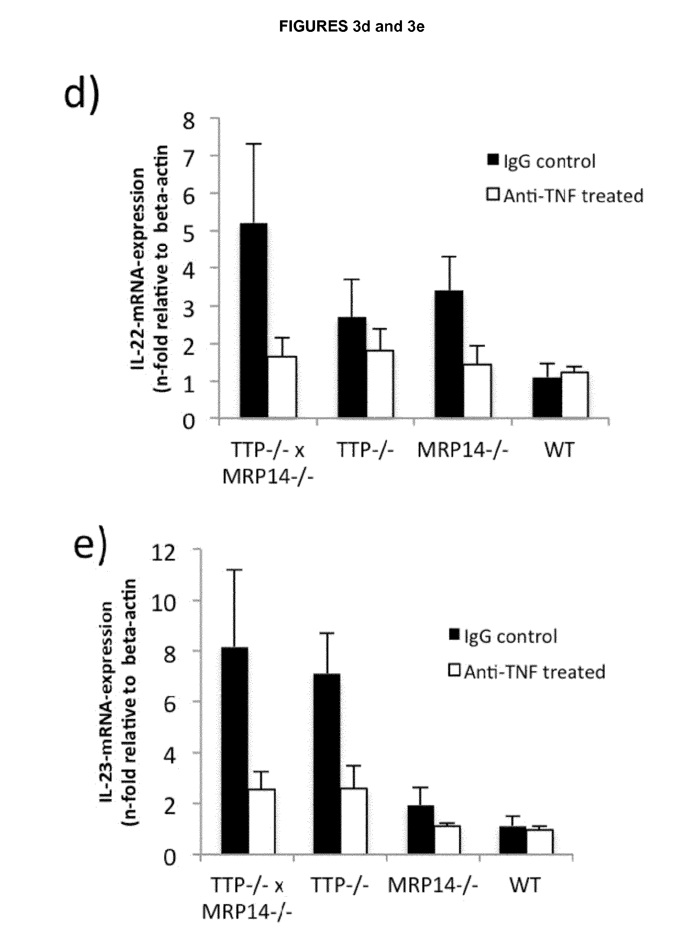

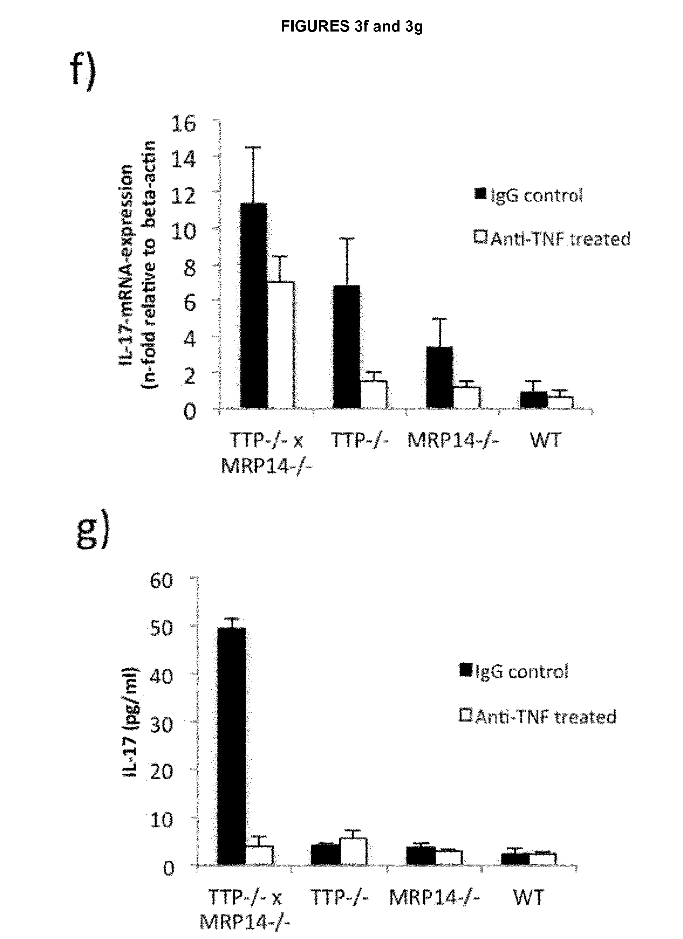

**Figure 3****:** Improvement of psoriasis-like phenotypes of the skin in TTP_{-/-} x MRP14_{-/-} mice by treatment with anti-TNF (e.g. Certolizumab). **3a,** Photographs and immunostainings of paraffin-embedded skin sections from anti-TNF (right panel) and IgG treated (left panel) TTP_{-/-} x MRP14_{-/-} mice at day 9. Sections were stained with H&E or with antibodies against the epidermal differentiation markers K14, K10 and Lor, or of frozen skin with antibodies against the immune cell markers F4/80, Gr1 and CD3 (green signal) or with antibodies against MRP8 (red signal). Red counterstaining shows tissue structure. Scale bar: 200 µm (H&E); 50 µm (immunostaining). **3b,** Adapted PASI score of anti-TNF and IgG control treated TTP_{-/-} x MRP14_{-/-} mice **c,** Dot blot analysis showing MRP8 expression in skin of anti-TNF and IgG treated TTP_{-/-} x MRP14_{-/-} mice relative to WT. **3d-3f,** mRNA levels of IL-17, IL-23 and IL-22 in the skin of anti-TNF and IgG treated TTP_{-/-} x MRP14_{-/-} mice were measured by real-time quantitative PCR. **3g,** IL-17 elisa of skin samples from anti-TNF and IgG treated TTP_{-/-} x MRP14_{-/-} mice.

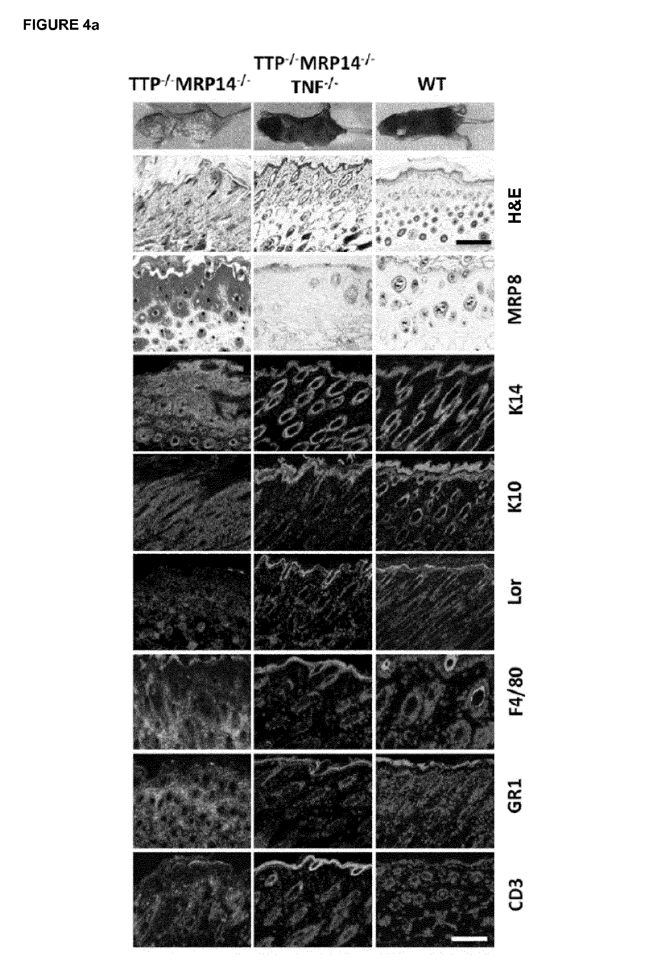

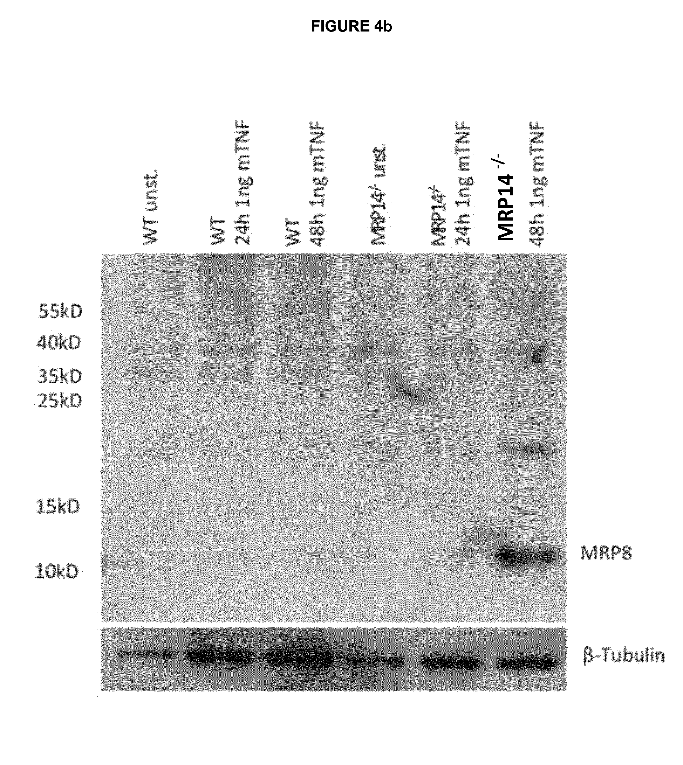

**Figure 4****:** Knockout of TNF protects TTP_{-/-} x MRP14_{-/-} mice from psoriasis-like phenotypes. **4a,** TTP_{-/-} x MRP14_{-/-} x TNF-/- mice do not develop inflammatory skin disease. (top panel). Skin sections were stained with H&E or MRP8 (red) or with antibodies against the indicated differentiation or immune cell markers (green signal). Nuclei are in red. Scale bar: 200 µm (H&E); 50 µm (immunostaining). **4b,** Western blot analysis showing expression of MRP8 and β-Tubulin in untreated and murine-TNF treated WT- and MRP14_{-/-} keratinocytes isolated from mice with the indicated genotypes.

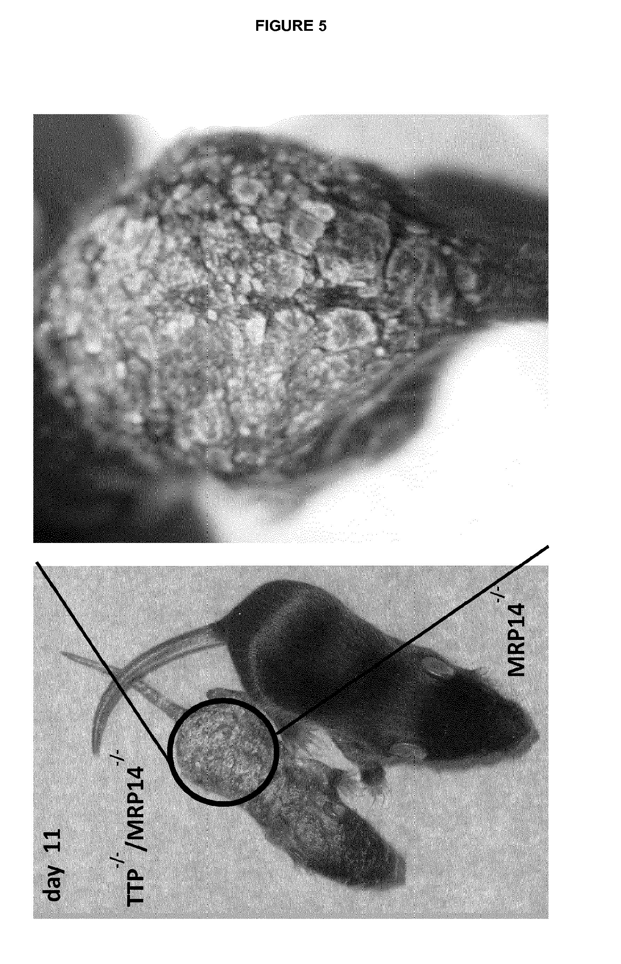

**Figure 5****:** TTP-/- x MRP14-/- and control mouse at day 11. The TTP-/- x MRP14-/- mouse exhibits a highly rigid shell-like skin with widespread scaling and it's growth is retarded.

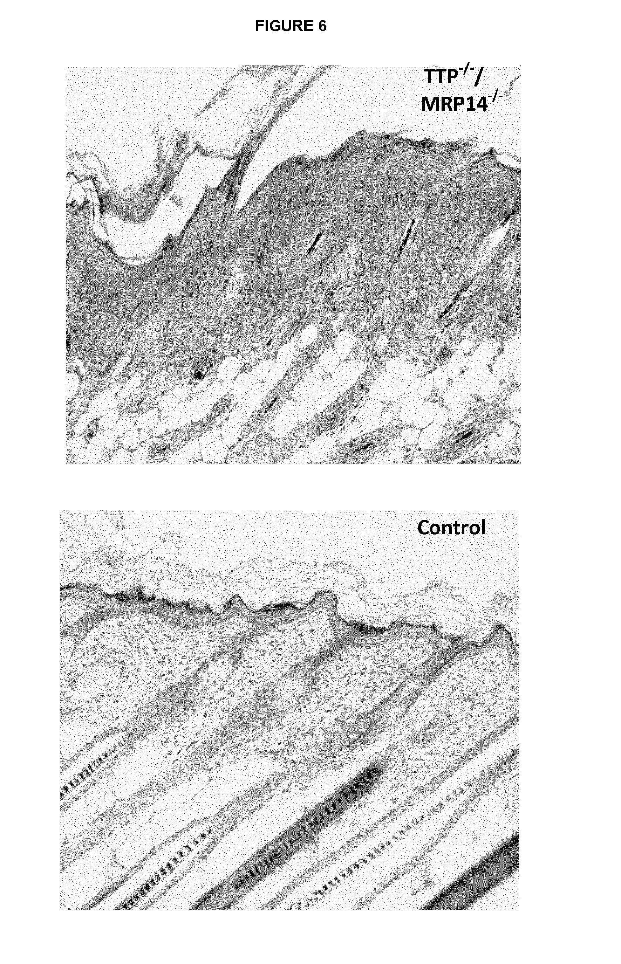

**Figure 6****:** Inflammation in the skin of TTP-/- x MRP-/- mice (day 9). Mice were stained with haematoxylin/eosin (H&E). TTP-/- x MRP-/- mice revealed a markedly thickened epidermis with loss of the granular layer, pronounced hyperkeratosis, focal parakeratosis and increased cellularity in the dermis.

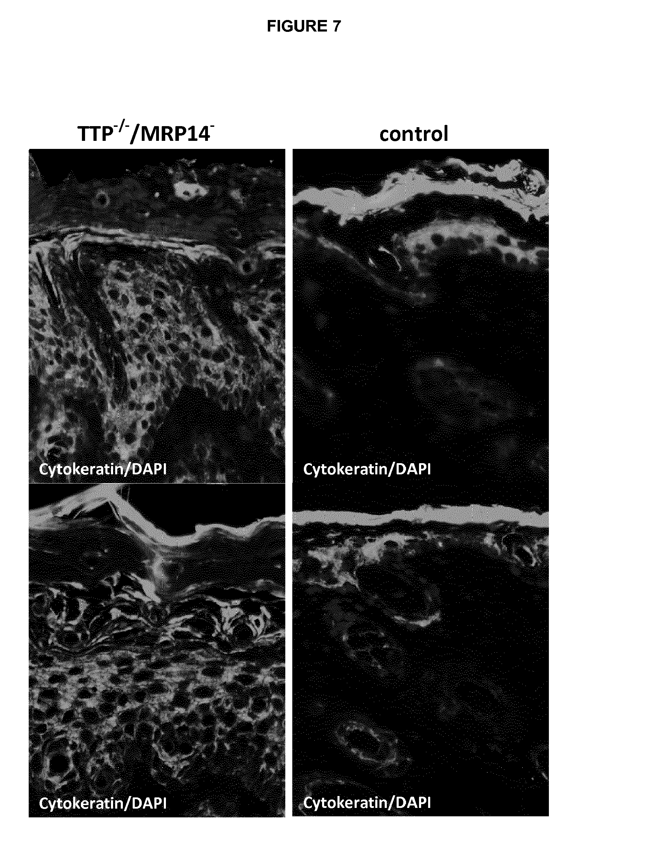

**Figure 7****:** Immunostaining. Cytokeratin staining of TTP-/- x MRP14-/- mice. The staining of the cytokeratin in the hyperproliferative epidermis is an additional Marker for the psoriatic phenotype.

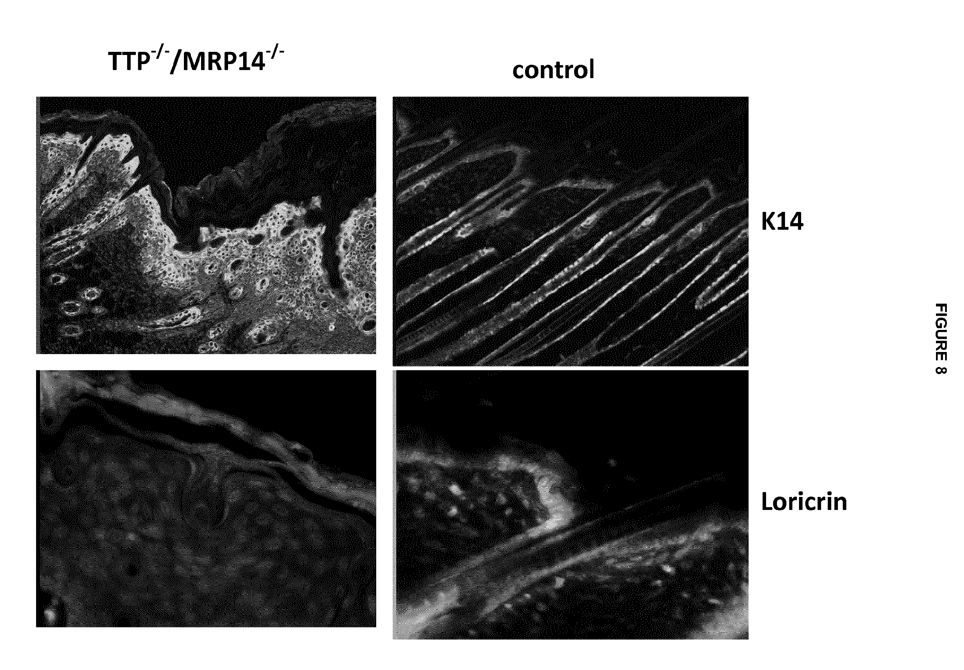

**Figure 8****:** Immunostaining. Histology of TTP-/- x MRP14-/- and control mice immunostained for Keratin 14 and Loricrin. The TTP-/- x MRP14-/- mice show subrabasal expression of K14 which is normally confined to the basal epidermal layer. In contrast the kerationocyte terminal differentiation marker loricrin was markedly reduced.

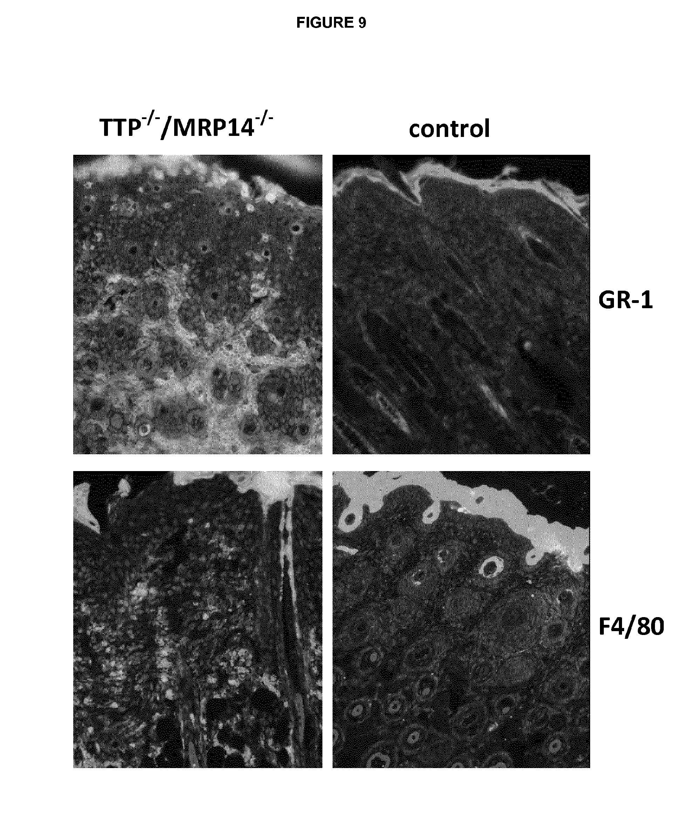

**Figure 9****:** Immunostaining. Inflammation in the skin of TTP-/- x MRP14-/-. Sections were immunostained with antibodies recognizing granulocytes (Gr-1) and macrophages (F4/80). TTP-/- x MRP14-/- show a high increase of invading granulocytes and macrophages in the dermis which is typical for psoriatic skin.

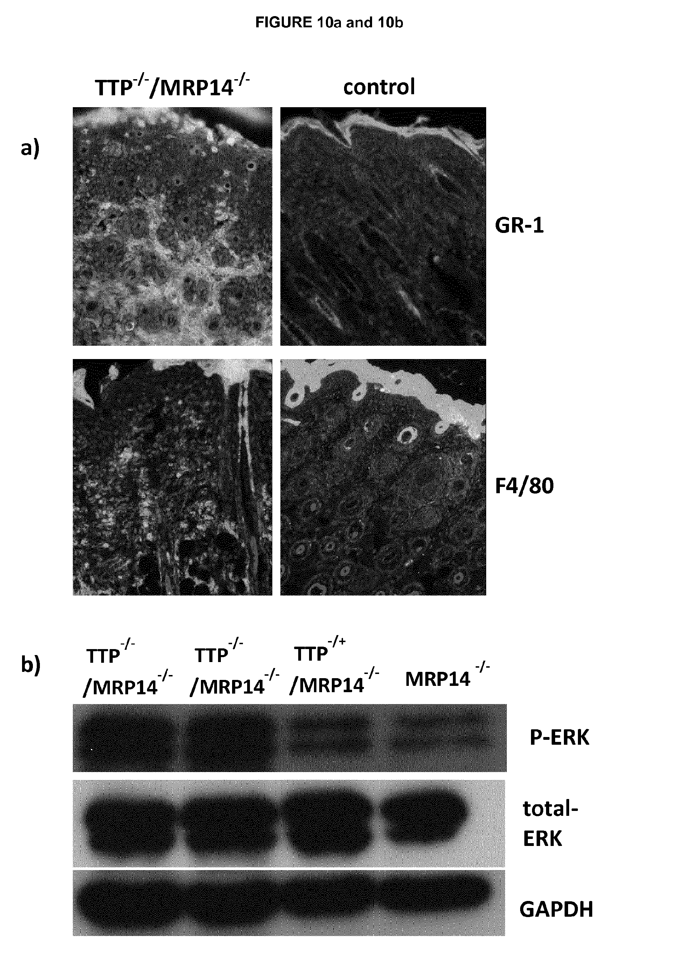

**Figure 10: 10a****,** see Fig 9. **10b****,** Western blot analysis of ERK phosphorylation of skin samples from TTP-/- x MRP14-/- and control mice. The upper panel shows phosphorylated RRK (pERK), and the middle panel shows total ERK and the bottom panel GAPDH as loading controls. The western blot revealed a strong nuclear signal for phosphorylated ERK in TTP-/- x MRP14-/- mice and therefore an activation of the ERK-dependent signalling pathway in the skin. ERK activation in the skin is an additional marker for the disturbed proliferation and/or differentiation of keratinocytes.

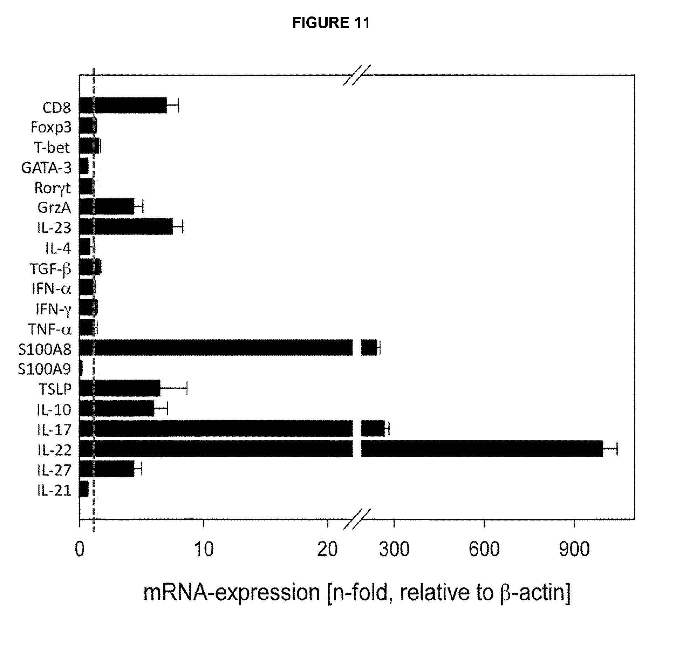

**Figure 11****:** Real-time quantitative PCR analysis. Expression in healthy control mice = 1. Shown is the n-fold-upregulation in diseased animals compared to controls. n = 4 diseased mice.

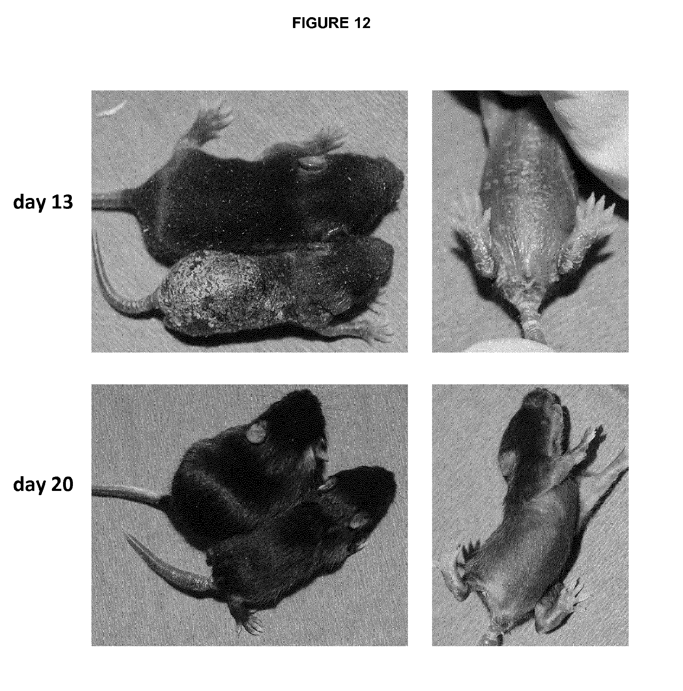

**Figure 12****:** Photos of TTP-/- x MRP14-/- and control mice from the same litter (left panel) and TTP-/- x MRP14-/- mouse (right panel) during Anti-TNF treatment at day 13 and 20. The Anti-TNF treatment led to a remission/suppression of the psoriatic phenotype.

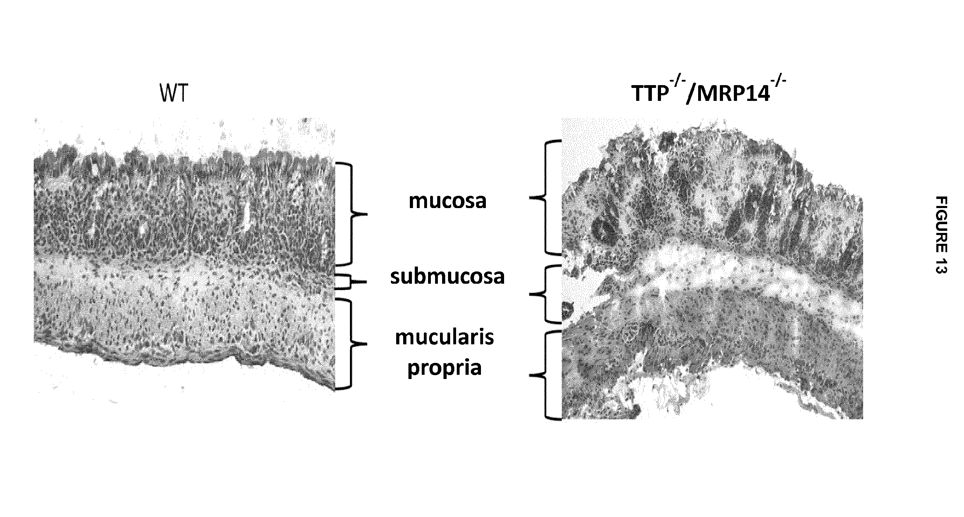

**Figure 13****:** Hematoxilin/Eosin (H&E) staining of colon sections from TTP-/- x MRP14-/- and WT mice. TTP-/- x MRP14-/- mice showed a strong effected epithelial layer with ulcerations and loss of the colon crypt structure. The muscularis propria in the enlarged submocosa and mucosa was infiltrated with inflammatory cells. There were also edematous distensions. This shows that the loss of MRP14 leads not only to a severe psoriatic phenotype but also to an inflammatory intestinal disease.

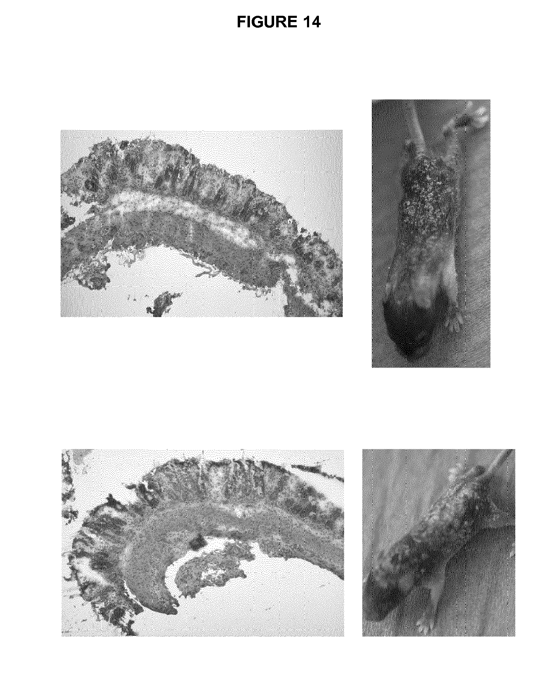

**Figure 14****:** Hematoxilin/Eosin (H&E) staining of colon sections from TTP-/- x MRP14-/- mice (left panel) and the corresponding photo of the mice (right panel). See Fig. 13 and Fig. 5.

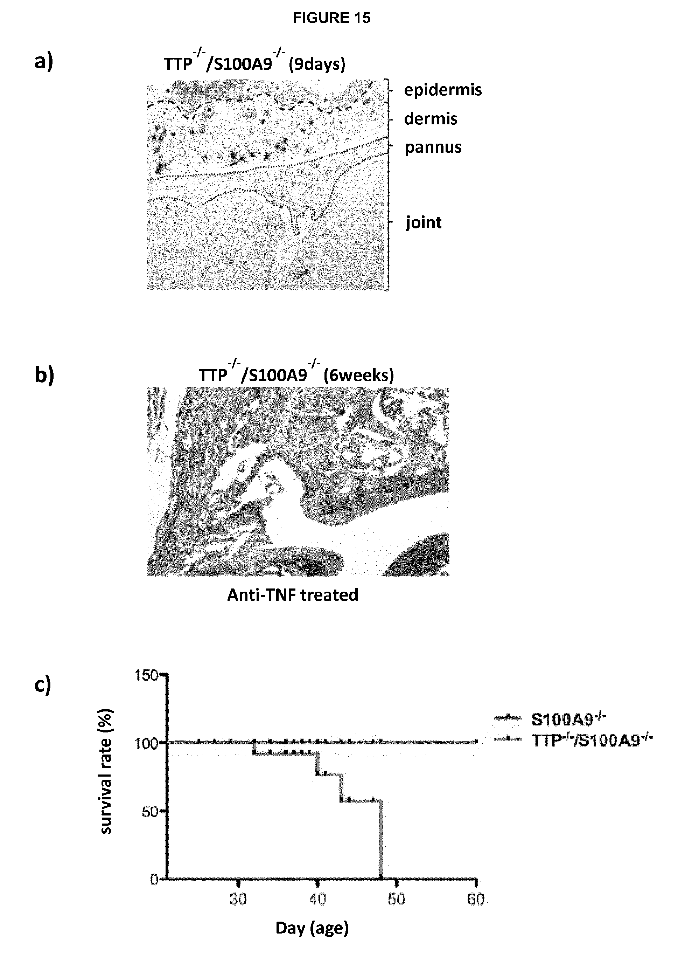

**Figure 15:** a) Immunohistochemistry. Histochemical MRP8 staining of the Hind paw from a 9 days old TTP_{-/-} x MRP14-/- mouse. The staining shows an overexpression of MRP8 in the hyperproliferative Epidermis. MRP8 is also detected in immuncells of the dermis and the invading pannus to the intra articular space. b) The Toluidine blue staining reveals despite anti-TNF treatment a clearly accelerated progress of the arthritic phenotype in TTP-/- x MRP14 (6 weeks) compared to TTP-/- mice (16-28 weeks). The cartilage is distained (loss of proteoglycan) and the arrows mark the invading pannus and bone destruction. c) Survival rate of TTP-/- x MRP14 mice after Anti-TNF treatment. TTP-/- x MRP14 mice died from day 30 to 50 after treatment completion.

### DETAILED DESCRIPTION OF THE INVENTION

Unless otherwise stated, the following terms used in this document, including the description and claims, have the definitions given below.

Those skilled in the art will recognize, or be able to ascertain, using not more than routine experimentation, many equivalents to the specific embodiments of the invention described herein. Such equivalents are intended to be encompassed by the present invention.

Unless otherwise defined herein, scientific and technical terms used in connection with the present invention shall have the meanings that are commonly understood by those of ordinary skill in the art. The methods and techniques of the present invention are generally performed according to conventional methods well known in the art. Generally, nomenclatures used in connection with, and techniques of biochemistry, enzymology, molecular and cellular biology, microbiology, genetics and protein and nucleic acid chemistry and hybridization described herein are those well-known and commonly used in the art.

Unless otherwise required by context, singular terms shall include pluralities and plural terms shall include the singular. Furthermore it is to be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series.

Several documents are cited throughout the text of this disclosure. Each of the documents cited herein (including all patents, patent applications, scientific publications, manufacturer's specifications, instructions, etc.), whether supra or infra, are hereby incorporated by reference in their entirety. To the extent the material incorporated by reference contradicts or is inconsistent with this specification, the specification will supersede any such material. Nothing herein is to be construed as an admission that the invention is not entitled to antedate such disclosure by virtue of prior invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having". When used herein "consisting of" excludes any element, step, or ingredient not specified in the claim element. In each instance herein any of the terms "comprising" and "consisting of" may be replaced with either of the other two terms.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or" as used herein.

The word "about" as used herein refers to a value being within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. The term "about" is also used to indicate that the amount or value in question may be the value designated or some other value that is approximately the same. The phrase is intended to convey that similar values promote equivalent results or effects according to the invention. In this context "about" may refer to a range above and/or below of up to 10%.

As described herein, "embodiment(s)" and "aspect(s)" means "embodiment(s) of the present invention" or "aspect(s) of the present invention", respectively.

The terms "of the invention" and "of the present invention" are used interchangeably herein.

The terms "non-human transgenic double knockout animal", "non-human transgenic double knockout animal of the invention", "non-human transgenic animal", "non-human transgenic animal of the invention", "non-human animal" and "non-human animal of the invention" are used interchangeably herein.

The present invention is, at least partly, based on the surprising finding that transgenic mice, in which the gene encoding Tristetraprolin (TTP) and the gene encoding myeloid-related protein-14 (MRP14; S100A9) are knocked out, develop severe psoriasis-like phenotypes.

Mice carrying a single knockout of the gene encoding TTP (TTP^{-/-} mice) were used before as a systemic autoimmune inflammatory model. TTP^{-/-} mice exhibit an increased stability of TNFalpha (TNF) mRNA and consequently higher levels of the cytokine at protein level. TTP^{-/-} mice appear normal at birth and after months they develop marked medullary and extramedullary myeloid hyperplasia associated with cachexia, erosive arthritis, conjunctivitis and dermatitis (Taylor et al., 1996).

Likewise, mice carrying a single knockout of the gene encoding MRP14 (MRP14^{-/-} mice) have been investigated before (e.g. Manitz et al., 2003). MRP14^{-/-} mice are healthy and develop normally. The protein MRP14 is expressed at very low levels in normal skin and is known to become **upregulated** during psoriasis (Broome et al., 2003; Mirmohammadsadegh et al., 2000; Quekenborn-Trinquet et al., 2005).

The discovery of the present inventors, that mice **lacking** MRP14 and TTP actually develop hallmarks of psoriasis was therefore completely unforeseeable. Even more surprisingly was that mice carrying a double knockout of TTP and MRP14 are an exceptionally good animal model for human psoriasis since these mice reflect multiple major symptoms of human patients suffering from psoriasis. TTP^{-/-}/MRP14^{-/-} mice develop all histological characteristics of human psoriasis, mimic psoriatic arthritis and exhibit alterations of the intestine characteristic for human psoriasis (and inflammatory diseases of the bowel). In particular TTP^{-/-}/MRP14^{-/-} mice have in common with human psoriasis the following clinical and histopathological features: hard, inflamed, inflexible skin with widespread scaling; thickening of the epidermis (acanthosis); thickening of the cornified layer (hyperkeratosis); retention of nuclei in the cells of the stratum corneum of the epidermis (parakeratosis); suprabasal expression of keratin-14; hyperproliferation of the epidermis; reduced expression of keratin-10 and loricrin in the skin; incomplete keratinocyte-differentiation; immune cell invasion in the dermis including accumulation of macrophages, granulocytes and CD3-positive T cells; increased expression of IL-17, IL-22 and IL-23. Moreover TTP^{-/-}/MRP14^{-/-} mice exhibit symptoms of inflammatory diseases of the bowel like epithelial denudation of the intestine; altered intestinal crypts; an increased number of inflammatory infiltrates, submucosal edema and increased number of ulcers in intestine.

Is sum, the present invention provides a TTP/MRP double knockout model animal, means of producing this animal and uses of said animal. Moreover the present invention relates to tissues, isolated cell(s) and cell lines deficient in TTP and MRP14 and uses thereof. These and further aspects and embodiments of the present invention are in the following characterized in detail.

In a fist aspect, the present invention provides a non-human transgenic double knockout animal, in which the following two genes are knocked out, gene encoding Tristetraprolin (TTP) and gene encoding myeloid-related protein-14 (Mrp14; S100A9). Said animal is referred to herein as "non-human transgenic animal of the invention". In addition, the term "non-human transgenic animal of the invention" includes all embodiments which further characterize the non-human transgenic animal of the invention as defined above. Moreover "non-human transgenic animal" includes the founder non-human transgenic animals of the invention and progeny of the founders. If not otherwise defined herein, "non-human transgenic animal of the invention" also includes cells, cell lines and tissues derived from the non-human transgenic animal of the invention.

As used herein "TTP" preferably refers to the protein Tristetraprolin. Alternative names of Tristetraprolin include growth factor-inducible nuclear protein NUP475, protein TIS11A (TIS11), TPA-induced sequence 11 and Zinc finger protein 36 (Zfp-36). These names can be used interchangeably herein. In the term "TTP-/-" or "TTP-/+" the letters "TTP" refer to the gene encoding the protein Tristetraprolin. "TTP-/-" refers to a homozygous knockout of the gene encoding TTP. "TTP^{-/+}" refers to a heterozygous knockout of the gene encoding TTP. "TTP" and *"ttp"* are to be understood as synonymous.

In preferred embodiments, the non-human transgenic animal of the present invention is a mammal or a fish, more specifically a zebrafish or a rodent such as a mouse, rat, rabbit, or guinea pig. Preferably "TTP" refers herein to the protein Tristetraprolin of a rodent such as a mouse, rat, rabbit, or guinea pig. Within the present invention, the TTP protein has typically an amino acid sequence that is homologous to the amino acid sequence of Tristetraprolin of a mammal, such as mouse Tristetraprolin, rat Tristetraprolin, rabbit Tristetraprolin, guinea pig Tristetraprolin as set forth in the common protein and/or gene databases like e.g. Uniprot database, NCBI databases (like UniGene or UniPept) and/or ENSEMBL database. The present invention also includes that the TTP protein can have one or more conservative or non-conservative amino acid substitutions, or additions or deletions from the TTP amino acid sequence (of the particular species) as set forth in the common protein and/or gene databases like e.g. Uniprot, NCBI or ENSEMBL databases. "Set forth" refers to a database search including the use of all the above listed alternative names of TTP.

In particular embodiments of the present invention the TTP protein has an amino acid sequence that is identical to the amino acid sequence set forth in GenPept Accession No.: NP_035886.1. In another embodiment of the present invention the TTP protein has an amino acid sequence that is at least 60 %, at least 70 %, at least 75 %, at least 80 %, at least 85 %, at least 90 %, at least 95 % or at least 100 % identical to the amino acid sequence set forth in GenPept Accession No.: NP_035886.1.

As used herein, "gene encoding TTP" refers to a gene that encodes TTP as defined herein. The term "gene encoding TTP" is understood to include the various sequence polymorphisms and allelic variations that exist within a population. This term relates primarily to an isolated coding sequence, but can also include some or all of the flanking regulatory elements and/or intron sequences (e.g., genomic sequence). "Gene encoding TTP" preferably refers herein to the nucleotide sequence of the gene encoding Tristetraprolin of a particular species, e.g. mouse, rat, rabbit, guinea pig, or zebrafish, as set forth in the common protein and/or gene databases like e.g. Uniprot, NCBI or ENSEMBL databases.

In embodiments in which the non-human animal is a mouse, "gene encoding TTP" refers to the gene Zfp-36, which is e.g. described in detail on www.ensembl.org (ENSEMBL Reference Sequence: ENSMUSG00000044786) and/or www.uniprot.org (Uniprot Entry No: P22893). The mouse gene encoding TTP (Zfp-36) is transcribed into a mRNA, which contains 1765 nucleotides (GenBank Accession No.: NM_011756.4). In embodiments in which the non-human animal is a mouse, the TTP protein preferably has an amino acid sequence that is homologous to the amino acid sequence set forth in GenPept Accession No. NP_035886.1.. It is envisaged that the TTP protein can have one or more conservative or non-conservative amino acid substitutions, or additions or deletions from the TTP amino acid sequence as set forth GenPept Accession No. NP_035886.1..

As used herein "MRP14" or "S100A9" preferably refers to the protein myeloid-related protein-14. Alternative names of MRP14 include calgranulin-B, leukocyte L1 complex heavy chain, migration inhibitory factor-related protein 14 (MRP14; p14), and S100 calcium-binding protein A9 (S100A9). These names can be used interchangeably herein. In the term "MRP14^{-/-}" or "MRP14^{-/+}" the letters "MRP14" refer to the gene encoding the protein myeloid-related protein-14. "MRP14^{-/-}" refers to a homozygous knockout of the gene encoding MRP14. "MRP14^{-/+}" refers to a heterozygous knockout of the gene encoding MRP14. "MRP14" and *"mrp14"* are to be understood as synonymous.

In preferred embodiments, the non-human transgenic animal of the present invention is a mammal or a fish, more specifically a zebrafish or a rodent such as a mouse, rat, rabbit, or guinea pig. Preferably "MRP14" refers herein to the protein MRP14 of a rodent such as a mouse, rat, rabbit, or guinea pig. Within the present invention the MRP14 protein has typically an amino acid sequence that is homologous to the amino acid sequence of MRP14 of a mammal, such as mouse MRP14, rat MRP14, rabbit MRP14, guinea pig MRP14, as set forth in the common protein and/or gene databases like e.g. Uniprot database, NCBI databases (like UniGene or UniPept) and/or ENSEMBL database. The present invention also includes that the MRP14 protein can have one or more conservative or non-conservative amino acid substitutions, or additions or deletions from the MRP14 amino acid sequence (of the particular species) as set forth in the common protein and/or gene databases like e.g. Uniprot, NCBI or ENSEMBL databases. "Set forth" refers to a database search including the use of all the above listed alternative names of MRP14, especially S100A9.

In particular embodiments of the present invention the MRP14 protein has an amino acid sequence that is identical to the amino acid sequence set forth in GenPept Accession No.: NP_033140.1. In another embodiment the MRP14 protein has an amino acid sequence that is at least 60 %, at least 70 %, at least 75 %, at least 80 %, at least 85 %, at least 90 %, at least 95 % or at least 100 % identical to the amino acid sequence set forth in GenPept Accession No.: NP_033140.1.

As used herein, "gene encoding MRP14" refers to a gene that encodes MRP14 as defined herein. The term "gene encoding MRP14" is understood to include the various sequence polymorphisms and allelic variations that exist within a population. This term relates primarily to an isolated coding sequence, but can also include some or all of the flanking regulatory elements and/or intron sequences (e.g., genomic sequence). "Gene encoding MRP14" preferably refers herein to the nucleotide sequence of the gene encoding MRP14 of a particular species, e.g. mouse, rat, rabbit, guinea pig, or zebrafish, as set forth in the common protein and/or gene databases like e.g. Uniprot, NCBI or ENSEMBL databases.

In embodiments in which the non-human animal is a mouse, "gene encoding MRP14" refers to the gene S100a9, which is e.g. described in detail on www.ensembl.org (ENSEMBL Reference Sequence: ENSMUSG00000056071) and/or www.uniprot.org (Uniprot Entry No: P31725). The mouse gene encoding MRP14 (S100a9)) is transcribed into an mRNA, which contains 534 nucleotides (GenBank Accession No.: NM_009114.3). In embodiments in which the non-human animal is a mouse, the MRP14 protein preferably has an amino acid sequence that is homologous to the amino acid sequence set forth in GenPept Accession No. NP_033140.1. It is envisaged that the MRP14 protein can have one or more conservative or non-conservative amino acid substitutions, or additions or deletions from the MRP14 amino acid sequence as set forth GenPept Accession No. NP_033140.1.

The accession/entry numbers (e.g. GenBank Accession No., GenPept Accession No. and the like) cited herein, are not intended to be limiting. i.e. updated or former versions are also included.

Unless the context requires otherwise, the term "gene encoding TTP", gene encoding the protein TTP" and "endogenous gene encoding TTP" are used interchangeably herein. Unless the context requires otherwise, the term "gene encoding MRP14", gene encoding the protein MRP14" and "endogenous gene encoding MRP14" are used interchangeably herein.

The term "transgenic" as used herein refers to an organism, such as an animal or cell, which is genetically modified. As used herein "transgenic" means that said animal or cell has a genetic construction different from the wild type animal of that species or the corresponding wild type cell, respectively. Within the present invention "transgenic" primarily refers to an animal or cell(s) in which genome the gene encoding TTP and the gene encoding MRP14 are knocked out.

The term "knockout" as used herein means "gene knockout" and refers to a targeted partial, substantial, or complete deletion, silencing, inactivation, or down-regulation of a gene on a chromosome in a cell. Furthermore "knockout" refers to a partial or complete suppression of the expression of an endogenous protein in a cell, preferably the endogenous gene encoding TTP and the endogenous gene encoding MRP14. Likewise, "knockout" of a gene refers herein to an alteration or disruption in the sequence of a target gene (preferably gene encoding TTP or gene encoding MRP14, respectively), that results in a decrease of function of said target gene, preferably such that said target gene expression is undetectable or insignificant. The terms "disruption" and "alteration" refer to a partial or complete reduction in the expression and/or function of the gene encoding TTP or gene encoding MRP14.

A gene knockout as well as a double gene knockout can be accomplished by a variety of methods known to those skilled in the art. For example gene targeting using homologous recombination, mutagenesis (e.g., point mutation) and/or antisense technology could be used to generate a non-human transgenic animal of the invention. These methods are well known to the person skilled in the art and e.g. described herein and in WO2009142724, WO2012099983, and Hall et al., 2009, which are all incorporated herein by reference in their entirety.

"Gene targeting" for example refers to a type of homologous recombination which occurs as a consequence of the introduction of a knockout construct (e.g., vector) into a mammalian cell (e.g., an ES cell). "Knockout construct" thereby refers to a nucleic acid sequence that is designed to decrease or suppress the expression of a protein encoded by endogenous DNA sequences in a cell. "Knockout construct" also means an exogenous DNA which is integrated into the genome of a cell from which a transgenic animal develops and which remains in the genome of the mature animal, thereby suppressing the expression of an encoded gene product in one or more cell types or tissues of the transgenic animal.
The nucleic acid sequence used as the knockout construct is typically comprised of (1) DNA from some portion of the gene (exon sequence, intron sequence, and/or promoter sequence) to be suppressed and (2) a marker sequence used to detect the presence of the knockout construct in the cell. The knockout construct is inserted into a cell, and integrates with the genomic DNA of the cell in such a position so as to prevent or interrupt transcription of the native DNA sequence. Such insertion usually occurs by homologous recombination (i.e., regions of the knockout construct that are homologous to endogenous DNA sequences hybridize to each other when the knockout construct is inserted into the cell and recombine so that the knockout construct is incorporated into the corresponding position of the endogenous DNA). The knockout construct nucleic acid sequence may comprise 1) a full or partial sequence of one or more exons and/or introns of the gene to be suppressed, 2) a full or partial promoter sequence of the gene to be suppressed, or 3) combinations thereof.

Within the present invention, such a knockout construct is preferably inserted into an embryonic stem cell (ES cell) and is integrated into the ES cell genomic DNA, usually by the process of homologous recombination. This ES cell is then injected into, and integrates with, the developing embryo.

The term "ES cell" as used herein refers to pluripotent embryonic stem cells and to such pluripotent cells in the very early stages of embryonic development, including but not limited to cells in the blastocyst stage of development.

Introduction of a knockout construct into ES cells may be accomplished using a variety of methods well-known in the art, including, for example, electroporation, microinjection, and calcium phosphate treatment.

The term "double knockout" as used herein refers to a knockout of two genes (in the genome of an organism or isolated cell(s)), preferably the gene encoding TTP and the gene encoding MRP14. Within the present invention a double knockout of genes of a non-human transgenic animal or a cell can be achieved by standard gene knockout techniques known to those skilled in the art, as e.g. those described herein.

To achieve a double knockout of the endogenous gene encoding TTP (TTP gene) and the endogenous gene encoding MRP14 (MRP14 gene) both genes have to be knocked out, for example by one of the following strategies or a combination thereof:
a) complete deletion or partial deletion of the endogenous TTP gene and/or the endogenous MRP14 gene;
b) replacement of the endogenous TTP gene and/or the endogenous MRP14 gene, e.g. by an inactivated TTP gene or an inactivated MRP14 gene, respectively; and/or
c) disruption of the endogenous TTP gene and/or the endogenous MRP14 gene.

"Disruption of a gene" preferably refers to the insertion of a nucleic acid sequence into one region of the native DNA sequence (usually one or more exons) and/or the promoter region of a gene so as to decrease or prevent expression of that gene in the cell as compared to the wild type or naturally occurring sequence of the gene. By way of example, a nucleic acid construct can be prepared containing a DNA sequence encoding an antibiotic resistance gene (e.g. a neomycin-resistance gene) which is inserted into the DNA sequence that is complementary to the DNA sequence (e.g. promoter and/or coding region) to be disrupted. When this nucleic acid construct is then transfected into a cell, the construct will integrate into the genomic DNA. Thus, many progeny of the cell will no longer express the gene at least in some cells, or will express it at a decreased level, as the DNA is now disrupted by the antibiotic resistance gene.

In one embodiment the non-human transgenic double knockout animal of the invention, is a mouse, whose gene encoding TTP has been knocked out by the insertion of a neomycin resistance gene into the protein-coding region of the second exon of the gene encoding TTP, thereby introducing multiple stop codons upstream of the sequences encoding the two putative zinc fingers of TTP.

In another embodiment the non-human transgenic double knockout animal of the invention, is a mouse whose gene encoding MRP14 has been knocked out by the replacement of 760 bp of the genomic sequence containing exon 2 of the gene encoding MRP14 and the adjacent intron region with a pMC1-derived neomycin-poly(A) expression cassette, thereby deleting the initial start codon of the gene encoding MRP14.

In a particular embodiment the non-human transgenic double knockout animal of the invention, is a mouse that is characterized in that (i) its gene encoding TTP has been knocked out by the insertion of a neomycin resistance gene into the protein-coding region of the second exon of the gene encoding TTP, thereby introducing multiple stop codons upstream of the sequences encoding the two putative zinc fingers of TTP and (ii) its gene encoding MRP14 has been knocked out by the replacement of 760 bp of the genomic sequence containing exon 2 of the gene encoding MRP14 and the adjacent intron region with a pMC1-derived neomycin-poly(A) expression cassette, thereby deleting the initial start codon of the gene encoding MRP14.

Within the present invention the knockout of the endogenous gene encoding TTP and/or the endogenous gene encoding MRP14 can also be an inducible gene knockout. "Inducible gene knockout" means that the target gene of interest, herein the gene encoding TTP and the gene encoding MRP14, can be specifically inactivated at a given time point. In an inducible double knockout of the present invention both, the gene encoding TTP and the gene encoding MRP14 may comprise an inducible knockout or one of said genes is permanently knocked out while the other gene comprises an inducible knockout. An inducible knockout can be achieved for example by introducing an inducible or repressible promoter into the gene encoding TTP and/or the gene encoding MRP14, e.g. by use of a Tet-on or Tet-off system.

The term "gene" as used herein includes all coding and non-coding DNA regions of a gene and all regulatory regions functionally linked to said gene. This means that "gene" includes herein the open reading frame (ORF) and all exons, introns, 5'-untranslated regions of the DNA (5'-UTR), 3'-untranslated regions of the DNA (3'-UTR), the promoter and all regulatory elements regulating the transcription of the gene. As defined herein "regulatory element" or "transcriptional regulatory element" refers to any element involved in regulating the transcription of a nucleic acid molecule, preferably the gene encoding TTP and/or the gene encoding MRP14. As used herein transcriptional regulatory element is a nucleic acid and may act in "cis" or "trans". There are no limits on the distance at which the transcriptional regulatory element exerts its effect, e.g., it may act over a distance of 1-1000 kb. The transcriptional regulatory element can be an enhancer or repressor or may even act as both enhancer and repressor. An enhancer (also called activator) is defined herein as any nucleic acid molecule that increases transcription of a nucleic acid molecule when functionally linked to a promoter regardless of its relative position. A repressor (also called silencer) is defined as any nucleic acid molecule which inhibits the transcription when functionally linked to a promoter regardless of relative position. "Functionally linked" is to be understood broadly and means that there is an influential relationship between two or more nucleotide regions.

Within the present invention the knockout of the endogenous gene encoding TTP and/or the endogenous gene encoding MRP14 can also be a conditional gene knockout. "Conditional gene knockout" means that the target gene of interest, herein the gene encoding TTP and/or the gene encoding MRP14, is not knocked out in the whole body but just deactivated in one or more particular tissue(s), optionally in a time specific manner. Conditional knockout methods which could be used to generate a non-human transgenic animal of the invention are for example the Cre-Lox recombination system or the Flp-FRT recombination system. Methods to achieve a conditional gene knockout are known to those skilled in the art and e.g. described in WO2011055366 and WO2009132357.

By way of example, in the Cre-Lox recombination system the targeted gene region is sandwiched by IoxP sequences which are targeted by Cre recombinase. Animals with this gene locus are called flox animals or floxed animals. Typically, a flox animal is formed by transfecting embryonic stem cells, with a target vector carrying the target gene flanked by two LoxP sites. By crossing a flox animal with an animal expressing a Cre recombinase under the control of a tissue or time specific promoter, it is possible to delete the targeted gene only in cells of a specific tissue and/or only to a specific time. Alternatively, an animal/ES cell may be transfected with two expression constructs, one expressing the floxed gene of interest and the other expressing a Cre recombinase under the control of a tissue or time specific promoter.

Typically, two organism lines (e.g. two mice lines) are required for formation of a conditional knockout animal: a conventional transgenic line with, for example, Cre-targeted to a specific tissue or cell type, and a strain that embodies a target gene flanked by two recombination sites (e.g. LoxP sites) in a direct orientation ("floxed gene"). When the target gene is for example the gene encoding TTP (or MRP14), recombination occurs by excision and, consequently, inactivation of the floxed TTP (or MRP14) target gene. Since recombination and TTP (or MRP14) gene excision occurs only in those cells expressing Cre recombinase, the TTP (or MRP14) target gene remains active in all cells and tissues that do not express Cre recombinase. Gene excision is induced by a recombination activator, such as Poly I:C or interferon, which in turn triggers Cre recombinase expression. The recombination activator is preferably injected postnatally. The term "gene" preferably refers herein to a genomic sequence. However "gene" may also apply herein to a recombinant sequence, or a cDNA or mRNA. As used herein "gene" includes all forms of a gene, e.g. both alleles of a gene.

Within the present invention it is not excluded, that the non-human transgenic animal of the invention comprises further gene knockouts. For example the gene encoding Tumor necrosis factor alpha (TNF) can be additionally knocked out in the animal of the invention. Accordingly in one embodiment the present invention relates to a non-human transgenic animal, in which the following genes are knocked out: gene encoding TTP, gene encoding MRP14 and gene encoding TNF.

Diploid organisms have one copy of each gene (and therefore one allele) on each chromosome. If identical alleles of the gene are present on both chromosomes said organism is homozygous for this particular gene. If the alleles are different, the organism is heterozygous for this particular gene. The non-human double knockout animal of the present invention refers to a diploid organism. Accordingly the gene encoding TTP as well as the gene encoding MRP14 can be knocked out on both alleles (homozygous gene knockout; -/-) or just on one allele (heterozygous gene knockout; -/+).

'Homozygous knockout of the endogenous gene encoding TTP' or 'homozygous knockout of the endogenous gene encoding MRP14", means that the function of said gene has been substantially decreased so that expression of TTP or MRP14, respectively, is not detectable or only present at insignificant levels.

In a preferred embodiment the non-human transgenic animal of the invention is characterized in that the knockout of the gene encoding TTP is a homozygous knockout and the knockout of the gene encoding MRP14 is a homozygous knockout. In other words, in one embodiment the non-human transgenic animal of the invention is characterized in that both TTP alleles and both MRP14 alleles are knocked out. One could also say, in one embodiment the non-human transgenic animal of the invention is characterized in that the gene encoding TTP is knocked out on both chromosomes of the non-human transgenic animal of the invention and the gene encoding MRP14 is knocked out on both chromosomes of the non-human transgenic animal of the invention. These animals are referred herein to as "TTP^{-/-} x MRP14^{-/-}" or "TTP^{-/-} /MRP14^{-/-}". "TTP allele" refers to one copy of the gene encoding TTP on one chromosome. "TTP alleles" refers to both copies of the gene encoding TTP, the one on first chromosome and the one on the second chromosome. "MRP14 allele" refers to one copy of the gene encoding MRP14 on one chromosome. "MRP14 alleles" refers to both copies of the gene encoding MRP14, the one on first chromosome and the one on the second chromosome. The term "the gene encoding TTP/MRP14 is knocked out on both chromosomes" can be replaced by "the genes encoding TTP/MRP14 are knocked out on both chromosomes".

In another embodiment the non-human transgenic animal of the invention is characterized in that the knockout of the gene encoding TTP is a homozygous knockout and the knockout of the gene encoding MRP14 is a heterozygous knockout. In other words, in one embodiment the non-human transgenic animal of the invention is characterized in that both TTP alleles are knocked out and just one MRP14 allele is knocked out, wherein the other MRP14 allele comprises a functional gene encoding MRP14. One could also say, in one embodiment the non-human transgenic animal of the invention is characterized in that the gene encoding TTP is knocked on both chromosomes of the non-human transgenic animal of the invention and the gene encoding MRP14 is just knocked out on one chromosome of the non-human animal of the invention, wherein the other chromosome of the non-human animal of the invention comprises the gene encoding MRP14 in its native form. These animals are referred herein to as "TTP^{-/-} x MRP14^{-/+}" or "TTP^{-/-}/MRP14^{-/+}". "Native form" refers to the form of the gene that occurs in the genome of the corresponding wild type animal. In its native form the gene encoding MRP14 encodes functional MRP14, which is expressed in detectable levels. Likewise, the gene encoding TTP encodes in its native form functional TTP, which is expressed in detectable levels.

In another embodiment the non-human transgenic animal of the invention is characterized in that the knockout of the gene encoding TTP is a heterozygous knockout and the knockout of the gene encoding MRP14 is a homozygous knockout. In other words, in one embodiment the non-human transgenic animal of the invention is characterized in that both MRP14 alleles are knocked out and just one TTP allele is knocked out, wherein the other TTP allele comprises a functional gene encoding TTP. One could also say, in one embodiment the non-human transgenic animal of the invention is characterized in that the gene encoding MRP14 is knocked on both chromosomes of the non-human transgenic animal of the invention and the gene encoding TTP is just knocked out on one chromosome of the non-human animal of the invention, wherein the other chromosome of the non-human animal of the invention comprises the gene encoding TTP in its native form. These animals are referred herein to as "TTP^{-/+} x MRP14^{-/-}" or "TTP^{-/+}/MRP14^{-/-}".

In another embodiment the non-human transgenic animal of the invention is characterized in that the knockout of the gene encoding TTP is a heterozygous knockout and the knockout of the gene encoding MRP14 is a heterozygous knockout. In other words, in one embodiment the non-human transgenic animal of the invention is characterized in that just one TTP allele is knocked out, wherein the other TTP allele comprises a functional gene encoding TTP, and just one MRP14 allele is knocked out, wherein the other MRP14 allele comprises a functional gene encoding MRP14. One could also say, in one embodiment the non-human transgenic animal of the invention is characterized in that the gene encoding TTP is just knocked out on one chromosome of the non-human animal of the invention, wherein the other chromosome of the non-human animal of the invention comprises the gene encoding TTP in its native form and the gene encoding MRP14 is just knocked out on one chromosome of the non-human animal of the invention, wherein the other chromosome of the non-human animal of the invention comprises the gene encoding MRP14 in its native form. These animals are referred herein to as "TTP^{-/+} x MRP14^{-/+}" or "TTP^{-/+}/MRP14^{-/+}".

"Non-human transgenic animal of the invention" include animals comprising a homozygous or heterozygous gene knockout of the gene encoding TTP and animals comprising a homozygous or heterozygous gene knockout of the gene encoding MRP14. The non-human transgenic animal of the present invention may therefore exhibit a partially suppressed or completely missing expression of TTP and a partially suppressed or completely missing of the expression of MRP14. "Completely missing expression" as used herein means that the non-human transgenic animal of the present invention is, due to a homozygous gene knockout of TTP or MRP14, respectively, unable to produce detectable levels of TTP or MRP14, respectively.

Within the present invention a homozygous gene knockout of TTP (or MRP14, respectively) preferably results in an inability of the non-human transgenic animal carrying said gene knockout to produce detectable amounts of TTP (or MRP14, respectively). "Detectable" refers to standard techniques used for the detection of proteins (e.g. western blot analysis as explained in the examples herein). Since the knockdown efficiency of a gene can also be determined by quantifying the mRNA transcribed from the targeted gene, "detectable" also refers to standard techniques for quantifying mRNA e.g. real-time quantitative PCR as explained in the examples herein). "Detectable amounts" preferably refers to the amount of a protein in a whole cell lysate, as e.g. explained in the examples herein, or to the amount of a mRNA extracted from non-lysed cells or tissues, as e.g. explained in the examples herein.

Accordingly, in one embodiment the non-human transgenic double knockout animal of the invention lacks detectable amounts of the proteins TTP and MRP14. In another embodiment the non-human transgenic double knockout animal of the invention lacks detectable amounts of the mRNA encoding TTP and the mRNA encoding MRP14.

In another embodiment the non-human transgenic double knockout animal of the invention lacks detectable amounts of the protein TTP and exhibits a lower expression of the protein MRP14 in comparison to a corresponding wild type animal. In another embodiment the non-human transgenic double knockout animal of the invention lacks detectable amounts of the mRNA encoding TTP and exhibits a lower amount of the mRNA encoding MRP14 in comparison to a corresponding wild type animal.

In another embodiment the non-human transgenic double knockout animal of the invention lacks detectable amounts of the protein MRP14 and exhibits a lower expression of the protein TTP in comparison to a corresponding wild type animal. In another embodiment the non-human transgenic double knockout animal of the invention lacks detectable amounts of the mRNA encoding MRP14 and exhibits a lower amount of the mRNA encoding TTP in comparison to a corresponding wild type animal.

In another embodiment the non-human transgenic double knockout animal of the invention exhibits a lower expression of the protein TTP in comparison to a corresponding wild type animal and exhibits a lower expression of the protein TTP in comparison to a corresponding wild type animal. In another embodiment the non-human transgenic double knockout animal of the invention exhibits a lower amount of the mRNA encoding TTP in comparison to a corresponding wild type animal and exhibits a lower amount of the mRNA encoding TTP in comparison to a corresponding wild type animal

The terms "wild type" and "WT" refer herein to an organism (preferably an animal or cell) comprising no knocked out genes and carrying a functional gene encoding TTP and a functional gene encoding MRP14. "Wild type animal" and "corresponding wild type animal" are used interchangeable herein. "Corresponding" refers in this context to an animal of the same species, optionally to an animal of the same or a comparable age. Two animals are for example of a comparable age if they are in a similar stage of development. Animals of different genotypes may also be compared regardless of their age.

Typically the non-human transgenic animal of the present invention exhibits one or more phenotypes associated with at least one disease selected from the group consisting of psoriasis, psoriatic arthritis, psoriasis-like diseases or inflammatory diseases of the bowel.

The term "phenotype associated with psoriasis, psoriatic arthritis, psoriasis-like diseases and/or inflammatory diseases of the bowel" means that said phenotype of the animal of the invention reflects a pathologic alteration, characteristic hallmark and/or symptom of a human or animal subject who suffers from psoriasis, psoriatic arthritis, a psoriasis-like disease and/or an inflammatory disease of the bowel. "Reflects" means that the phenotype of the animal of the invention is equal or very similar to a pathologic alteration of a human or animal suffering from one or more of said diseases. "Reflects" does not exclude that there are minor differences between the phenotype of the animal of the invention and the pathologic alteration of a human or animal suffering from said diseases.

Pathological alterations of a human or animal suffering from psoriasis, psoriatic arthritis, a psoriasis-like disease and/or an inflammatory disease of the bowel are preferably those described herein as "phenotypes of the animal of the invention", e.g. acanthosis, hyperkeratosis, parakeratosis etc..

Preferably the term "psoriasis, psoriatic arthritis, psoriasis-like diseases and/or inflammatory diseases of the bowel" refers to human diseases. Accordingly, in some embodiments the non-human transgenic animal of the present invention exhibits at least one phenotype that reflects at least one disease selected from the group consisting of human psoriasis, human psoriatic arthritis, human psoriasis-like diseases or human inflammatory diseases of the bowel.

Within the present invention the term "psoriasis-like disease(s)" refers to conditions that cause similar symptoms as psoriasis. In particular psoriasis-like diseases refer to conditions that cause pathological alterations of the body, especially of the skin, which are characteristic for patients suffering from psoriasis. Said alterations include those described herein as "phenotypes of the animal of the invention". Psoriasis-like diseases as used herein include fungal and bacterial infections of the skin, allergic reactions of the skin, intertrigo, lichen planus, lupus erythematosus, skin cancer, eczema (e.g., contact dermatitis, atopic dermatitis, and nummular eczema), parapsoriasis, squamous cell carcinoma, lichen simplex chronicus, pityriasis alba, mycosis fungoides, oncychomycosis, seborrheic dermatitis, tinea and syphilis. Moreover "psoriasis-like diseases" as used herein includes alterations of the skin similar to psoriasis that are caused by medicaments. "Psoriasis-like diseases" as used herein furthermore includes psoriasis of non-human transgenic animals.

Within the present disclosure the terms "psoriasis-like disease(s)", "disease(s) associated with the development of psoriasis-like symptoms", "disease(s) associated with psoriasis-like symptoms" and "psoriasis like symptom(s)" may be replaced with either of the other terms. All these terms refer to pathological alterations of the body of a human or animal, especially of the skin, intestine or other organs, that are similar to symptoms associated with human psoriasis but caused by conditions different to psoriasis.

Within the present invention the term "inflammatory disease(s) of the bowel" includes Colitis ulcerosa, Crohn's disease, Collagenous colitis, Lymphocytic colitis, Ischaemic colitis, Diversion colitis, Behçet's disease, and colitis indeterminata.

Within the present invention "phenotypes associated with psoriasis, psoriatic arthritis, psoriasis-like diseases and/or inflammatory diseases of the bowel", "phenotype(s) as defined herein", "psoriatic phenotype(s) as defined herein" and "psoriatic phenotypes of the animal of the invention" preferably refer to the following phenotypes a) to s) which are defined below (and elsewhere herein) in more detail.

### Skin phenotypes:

a) acanthosis
b) hyperkeratosis
c) parakeratosis
d) increased suprabasal expression of Keratin 14 in the epidermis
e) reduced expression of keratin 10 in the stratum spinosum
f) reduced expression of loricrin in the stratum granulosum
g) increased number of macrophages, granulocytes and/or CD3-positive T-cells in the dermis and/or epidermis
h) increased activation of phosphorylated extracellular-signal regulated kinase (pERK)
i) increased amount of interleukin-17, interleukin-22 and/or interleukin-23 in the skin
j) hard, inflamed, inflexible skin with widespread scaling
k) incomplete keratinocyte-differentiation

### Enteric phenotypes:

l) epithelial denudation in the intestine
m) altered architecture of intestinal crypts
n) increased number of ulcers in the intestine
o) increased number of inflammatory infiltrates in the mucosa and or submucosa of the intestine
p) submucosal edema in the intestine

### Arthritic phenotypes:

q) bone erosions
r) loss of proteoglycans of cartilage
s) immigration of inflammatory infiltrate in the joint gap

Typically the non-human transgenic animal of the invention exhibits one or more phenotypes including, but not limited to, said phenotypes a) to s). Preferably the non-human transgenic animal of the invention exhibits more than 5 of said phenotypes a) to s). In particular the non-human transgenic animal of the invention exhibits at least one phenotype of the skin (skin phenotypes), at least one phenotype of the intestine (enteric phenotype) and/or at least one of the arthritic phenotypes.

The terms "exhibit", "exhibits" and "exhibiting" as used herein in the context of 'the non-human transgenic animal of the invention exhibits a phenotype' include that said animal develops said phenotype after birth (i.e. postnatal) in the course of further development. Accordingly, in one embodiment, the non-human transgenic animal of the invention develops a macroscopically observable expression of a phenotype associated with a disease selected from the group consisting of psoriasis, psoriatic arthritis, psoriasis-like diseases and/or inflammatory diseases of the bowel postnatally. Within the present invention the animal of the invention may therefore exhibit at birth macroscopically no differences compared to a wild type animal. "Macroscopically" means that it is possible for those skilled in the art to notice said phenotype by eye. It is encompassed by the present invention, that an animal of the invention may exhibits at least one phenotype of the phenotypes defined herein already at birth and/or develops said phenotype(s) postnatally. In the embodiments in which the non-human transgenic animal of the invention is a TTP^{-/-}/MRP14^{-/-} mouse, said mouse preferably shows no macroscopically observable differences compared to a corresponding wild type mouse until postnatal day 4 and develops at least one phenotype as defined herein until postnatal day 10.

In one embodiment the non-human transgenic animal of the invention exhibits the phenotype of acanthosis. It is within the scope of the invention that the non-human transgenic animal of the present invention exhibits acanthosis with loss of the granular layer. "Acanthosis" is a diffuse epidermal hyperplasia (thickening of the skin). Acanthosis is a hallmark of human psoriasis. In an embodiment the non-human transgenic animal of the invention is a TTP^{-/-} /MRP14^{-/-} mouse and, if desired, the presence of acanthosis can be determined by methods known to those skilled in the art e.g. at postnatal day 11.

"Methods known to those skilled in the art" as used herein are for instance those described in the present description and examples.

In the embodiments of the invention in which the non-human transgenic animal is not a mouse, the phenotypes described herein may be determined, according to the expertise of those skilled in the art, at a postnatal day that corresponds to the stage of development of a mouse on the postnatal days indicated herein for the embodiments in which the animal of the invention is a mouse.

In another embodiment the non-human transgenic animal of the invention exhibits the phenotype of hyperkeratosis. "Hyperkeratosis" is a thickening of the stratum corneum, often associated with a quantitative abnormality of the keratin, and also usually accompanied by an increase in the granular layer. As the corneum layer normally varies greatly in thickness in different sites, some experience is needed to assess minor degrees of hyperkeratosis. Hyperkeratosis is a hallmark of human psoriasis. In an embodiment the non-human transgenic animal of the invention is a TTP^{-/-}/MRP14^{-/-} mouse and, if desired, the presence of hyperkeratosis can be determined by methods known to those skilled in the art e.g. at postnatal day 11.

In another embodiment the non-human transgenic animal of the invention exhibits the phenotype of parakeratosis. Parakeratosis is a mode of keratinization characterized by the retention of nuclei in the stratum corneum. In mucous membranes, parakeratosis is normal. In the skin, this process leads to the abnormal replacement of annular squames with nucleated cells. Parakeratosis is usually associated with the thinning or loss of the granular layer and is seen in diseases of increased cell turnover, whether inflammatory or neoplastic. Parakeratosis is a hallmark of human psoriasis and in particular seen in the plaques of psoriasis. In an embodiment the non-human transgenic animal of the invention is a TTP^{-/-}/MRP14^{-/-} mouse and, if desired, the presence of parakeratosis can be determined by methods known to those skilled in the art e.g. at postnatal day 11.

In another embodiment the non-human transgenic animal of the invention exhibits the phenotype of an increased suprabasal expression of keratin-14 in the epidermis in comparison to a control. "Suprabasal" refers to the epidermal layers which are not the stratum basale. In particular "suprabasal" refers to the stratum spinosum, stratum granulosum and stratum corneum. Suprabasal expression of keratin-14 in the epidermis is a hallmark of human psoriasis. In an embodiment the non-human transgenic animal of the invention is a TTP^{-/-} /MRP14^{-/-} mouse and, if desired, the presence of a suprabasal expression of keratin-14 in the epidermis can be determined by methods known to those skilled in the art e.g. at postnatal day 9.

"Control" as used herein in the context of "in comparison to a control" preferably refers to a corresponding wild type animal, as defined above. In addition, "control" can also refer in this context to TTP single knockout animals and/or MRP14 single knockout animals. "Single knockout animal" refers herein to a non-human transgenic animal that comprises just one gene knockout. "TTP single knockout animal" refers herein to a non-human transgenic animal that comprises a knockout of the gene encoding TTP. Said knockout can be homozygous (TTP^{-/-} animals) or heterozygous (TTP^{-/+} animals). "MRP14 single knockout animal" refers herein to non-human transgenic animal that comprises a knockout of the gene encoding MRP14. Said knockout can be homozygous (MRP14^{-/-} animals) or heterozygous (MRP14^{-/+} animals). Beside the difference in the TTP gene and MRP14 gene, test and control animals preferably comprise the same genetic background.

In another embodiment the non-human transgenic animal of the invention exhibits the phenotype of a reduced expression of keratin-10 in the stratum spinosum in comparison to a control. Reduced expression of keratin-10 in the stratum spinosum is a hallmark of human psoriasis. In an embodiment the non-human transgenic animal of the invention is a TTP^{-/-} /MRP14^{-/-} mouse and, if desired, the presence of reduced expression of keratin 10 in the stratum spinosum can be determined by methods known to those skilled in the art e.g. at postnatal day 9.

In another embodiment the non-human transgenic animal of the invention exhibits the phenotype of a reduced expression of loricrin in the stratum granulosum in comparison to a control. A reduced expression of loricrin in the stratum granulosum is a hallmark of human psoriasis. In an embodiment the non-human transgenic animal of the invention is a TTP^{-/-} /MRP14^{-/-} mouse and, if desired, the presence of a reduced expression of loricrin in the stratum granulosum can be determined by methods known to those skilled in the art e.g. at postnatal day 9.

In another embodiment the non-human transgenic animal of the invention exhibits the phenotype of an increased number of macrophages, granulocytes and/or CD3-positive T-cells in the dermis and/or epidermis in comparison to a control. An increased number of macrophages, granulocytes and/or CD3-positive T-cells in the dermis and/or epidermis is a hallmark of human psoriasis. In an embodiment the non-human transgenic animal of the invention is a TTP^{-/-}/MRP14^{-/-} mouse and, if desired, the presence of an increased number of macrophages, granulocytes and/or CD3-positive T-cells in the dermis and/or epidermis can be determined by methods known to those skilled in the art e.g. at postnatal day 9.

It is also encompassed by the invention that the non-human transgenic animal of the invention exhibits an increased number of granulocytes under the stratum corneum within the epidermis in comparison to a control. In an embodiment the non-human transgenic animal of the invention is a TTP^{-/-}/MRP14^{-/-} mouse and, if desired, the presence of an increased number of granulocytes under the stratum corneum within the epidermis can be determined by methods known to those skilled in the art e.g. at postnatal day 9.

In another embodiment the non-human transgenic animal of the invention exhibits the phenotype of an increased activation of phosphorylated extracellular-signal regulated kinase (pERK) in the epidermis in comparison to a control. Activation of pERK is a hallmark of human psoriasis. In an embodiment the non-human transgenic animal of the invention is a TTP^{-/-} /MRP14^{-/-} mouse and, if desired, the presence of an increased activation of pERK in the epidermis can be determined by methods known to those skilled in the art e.g. at postnatal day 9.

In another embodiment the non-human transgenic animal of the invention exhibits the phenotype of an increased amount of interleukin-17, interleukin-22 and/or interleukin-23 in the skin in comparison to a control. An increased amount of interleukins in the skin is a hallmark of human psoriasis. In an embodiment the non-human transgenic animal of the invention is a TTP-^{/-}/MRP14^{-/-} mouse and, if desired, the presence of increased amount of interleukin-17, interleukin-22 and/or interleukin-23 in the skin can be determined by methods known to those skilled in the art e.g. at postnatal day 9.

In another embodiment the non-human transgenic animal of the invention exhibits an increased expression of myeloid-related protein-8 (MRP8; S100A8) in the epidermis in comparison to a control. MRP8 is expressed at very low levels in normal skin and becomes upregulated during psoriasis. In an embodiment the non-human transgenic animal of the invention is a TTP^{-/-}/MRP14^{-/-} mouse and, if desired, the presence of an increased expression of MRP8 in the epidermis can be determined by methods known to those skilled in the art e.g. at postnatal day 9.

It is also envisaged that the non-human transgenic animal of the invention exhibits a hard, inflamed, inflexible skin with widespread scaling. In an embodiment the non-human transgenic animal of the invention is a TTP^{-/-}/MRP14^{-/-} mouse which shows a hard, inflamed, inflexible skin with widespread scaling e.g. at postnatal day 9.

It is not excluded that also a control animal may exhibit a phenotype as described herein. However in the non-human transgenic animal of the invention the phenotypes described herein are preferably more pronounced and earlier developed in comparison to a control animal.

The non-human transgenic animal of the invention preferably has a higher adapted (Psoriasis Area and Severity Index) PASI-score than a control animal.

The on-human animal of the invention preferably exhibits at least one skin phenotype as described above and at least one enteric phenotype as described below.

In another embodiment the non-human transgenic animal of the invention exhibits epithelial denudation in the intestine, preferably in the colon. In an embodiment the non-human transgenic animal of the invention is a TTP^{-/-}/MRP14^{-/-} mouse and, if desired, the presence of epithelial denudation in the intestine can be determined by methods known to those skilled in the art e.g. at postnatal day 9.

In another embodiment the non-human transgenic animal of the invention exhibits an altered architecture of intestinal crypts, preferably in the colon, in comparison to a control. "Altered architecture of intestinal crypts" refers to elongated crypts, to a reduction of crypts and/or a complete destruction of crypts (lack of crypts) in comparison to a control. In an embodiment the non-human transgenic animal of the invention is a TTP^{-/-}/MRP14^{-/-} mouse and, if desired, the presence of an altered architecture of intestinal crypts can be determined by methods known to those skilled in the art e.g. at postnatal day 9.

In another embodiment the non-human transgenic animal of the invention exhibits an increased number of ulcers in the intestine, preferably in the colon, in comparison to a control. In an embodiment the non-human transgenic animal of the invention is a TTP^{-/-}/MRP14^{-/-} mouse and, if desired, the presence of an increased number of ulcers in the intestine can be determined by methods known to those skilled in the art e.g. at postnatal day 9.

In another embodiment the non-human transgenic animal of the invention exhibits an increased number of inflammatory infiltrates in the mucosa and/or submucosa of the intestine, preferably in the colon, in comparison to a control. In an embodiment the non-human transgenic animal of the invention is a TTP^{-/-}/MRP14^{-/-} mouse and, if desired, the presence of an increased number of inflammatory infiltrates in the mucosa and or submucosa of the intestine can be determined by methods known to those skilled in the art e.g. at postnatal day 9.

In another embodiment the non-human transgenic animal of the invention exhibits a submucosal edema in the intestine. In an embodiment the non-human transgenic animal of the invention is a TTP^{-/-}/MRP14^{-/-} mouse and, if desired, the presence of submucosal edemata in the intestine can be determined by methods known to those skilled in the art e.g. at postnatal day 9.

TTP is a posttranslational factor which regulates the posttranslational expression of Tumor necrosis factor alpha (TNF). The lack of TTP leads to an increased expression of TNF. Animals of the present invention, especially TTP^{-/-}/MRP14^{-/-}exhibit an increased level of TNF. As depicted in the examples herein, the inventors have demonstrated that psoriatic phenotypes of the animal of the invention depend on the TNF level. Suppression of TNF by gene knockout or anti-TNF therapy prevents and/or suppresses psoriatic phenotypes of the animal of the invention.

In one embodiment the non-human transgenic animal of the invention overexpresses TNF.

In another embodiment the present invention provides the non-human transgenic animal of the invention, wherein at least one psoriatic phenotype as defined herein is prevented and/or alleviated by suppressing or inhibiting TNF in said animal. Preferably TNF is suppressed by knocking out the gene encoding TNF or inhibited by subjecting the animal of the invention to an anti-TNF therapy. "Anti-TNF therapy" refers herein preferably to administering a TNF neutralizing antibody to the animal of the invention. A TNF neutralizing antibody is for example Certolizumab.

Thus, in a further embodiment the present invention relates to the non-human transgenic animal of the invention, wherein TNF is inhibited by subjecting an anti-TNF therapy to said animal. Preferably said anti-TNF therapy comprises administering a TNF neutralizing antibody to said animal, wherein said TNF neutralizing antibody is optionally Certolizumab.

The non-human transgenic double knockout animal of the invention is preferably a non-human mammal or a fish. In preferred embodment the non-human animal of the invention is a rodent. In some embodiments the non-human transgenic animal of the invention, is a mouse, rat, rabbit, guinea pig or zebrafish. In one embodiment the non-human transgenic animal of the invention is a mouse. In particular embodiments the non-human transgenic animal of the invention is a TTP^{-/-}/MRP14^{-/-} mouse, TTP^{-/+}/MRP14^{-/-} mouse, TTP^{-/-}/MRP14^{-/+} mouse or TTP^{-/+}/MRP14^{-/+} mouse. In a preferred embodiment the non-human transgenic animal of the invention is a TTP^{-/-}/MRP14^{-/-} mouse, TTP^{-/+}/MRP14^{-/-} mouse or TTP^{-/+}/MRP14^{-/+} mouse. In another preferred embodiment the non-human transgenic animal of the invention is a TTP^{-/-} /MRP14^{-/-} mouse or TTP^{-/+}/MRP14^{-/-} mouse mouse. In a further preferred embodiment the non-human transgenic animal of the invention is a TTP^{-/-}/MRP14^{-/-} mouse.

The non-human transgenic animal of the invention represents an animal model suitable for analyzing molecular mechanisms underlying psoriasis, psoriatic arthritis, psoriasis-like diseases and/or inflammatory diseases of the bowel. Accordingly, in one embodiment the non-human transgenic animal of the invention is for use in identifying and/or characterizing mechanisms underlying psoriasis, psoriatic arthritis, psoriasis-like diseases and/or inflammatory diseases of the bowel. Likewise, the non-human transgenic animal of the invention is an animal model for use in identifying and/or characterizing molecular and cellular mechanisms underlying psoriasis, psoriatic arthritis, psoriasis-like diseases and/or inflammatory diseases of the bowel.

In addition, the non-human transgenic animal of the invention represents an animal model suitable for identifying and/or testing compounds for use in the therapy of a disease selected from the group consisting of psoriasis, psoriatic arthritis, psoriasis-like diseases and/or inflammatory diseases of the bowel. "Therapy" as used herein preferably refers to prevention, treatment and/or alleviation of a disease or pathological condition. "Compound" includes therapeutic agents as well as pharmaceutical compositions comprising a therapeutic agent. As used herein the term "testing" includes e.g. determining therapeutically effective amounts of a compound, determining optimal administration intervals and/or determining appropriate combinations of pharmaceutical excipients and therapeutic agent(s) in a pharmaceutical composition. Accordingly, in another embodiment the non-human transgenic animal of the invention is for use in identifying and/or testing compounds for use in the prevention, treatment or alleviation of a disease selected from the group consisting of psoriasis, psoriatic arthritis, psoriasis-like diseases and inflammatory diseases of the bowel. Likewise, the non-human transgenic animal of the invention is an animal model for use in identifying and/or testing compounds for use in the prevention, treatment or alleviation of a disease selected from the group consisting of psoriasis, psoriatic arthritis, psoriasis-like diseases and inflammatory diseases of the bowel.

In a further embodiment the non-human transgenic animal of the invention is for use in the development of a method for the diagnosis of a disease selected from the group consisting of psoriasis, psoriatic arthritis, psoriasis-like diseases and inflammatory disease of the bowel. Likewise, the non-human transgenic animal of the invention is an animal model for use in the development of a method for the diagnosis of a disease selected from the group consisting of psoriasis, psoriatic arthritis, psoriasis-like diseases and/or inflammatory diseases of the bowel.

In a second aspect, the present invention refers to a method for generating the non-human transgenic animal of the invention as defined above. Non-human transgenic animals of the invention can be obtained by genetically modifying a non-transgenic animal, in that way that (i) the endogenous gene encoding TTP of said animal is knocked out on either one or both TTP alleles, and (ii) the endogenous gene encoding MRP14 of said animal is knocked out on either one or both MRP14 alleles. Non-human transgenic animals of the invention can be generated by various methods known to those skilled in the art. It is to be understood that these methods are encompassed by the invention.

Animals in which the gene encoding TTP is knocked out on both alleles (TTP^{-/-}/MRP14^{-/-} animals or TTP^{-/-}/MRP14^{-/+} animals) exhibit decreased fertility. Accordingly, for breeding the non-human transgenic animal of the invention it is preferred to mate animals in which just one TTP allele is knocked out, wherein the other TTP allele comprises a functional gene encoding TTP (TTP^{-/+}/MRP14^{-/-} animals or TTP^{-/+}/MRP14^{-/+} animals). Preferably non-human transgenic animals of the invention are bred by mating TTP^{-/+}/MRP14^{-/-} animals.

TTP^{-/-}/MRP14^{-/-} animals are preferably used for experiments.

In a particular embodiment, the present invention provides a method to generate the animal of the invention, wherein said animal is a mouse and the method for generating said mouse comprises
(i) crossing MRP14^{-/-} mice or MRP14^{-/+} mice with TTP^{-/+} mice;
(ii) genotyping of mice obtained in step (i) e.g. by PCR from tail DNA;
(iii) backcrossing of TTP^{-/+}/MRP14^{-/+} mice into one of the genetic background mouse strains, preferably for at least 10 generations; and
(iv) genotyping of mice obtained in step (iii) e.g. by PCR from tail DNA.

TTP single knockout mice which can be used to generate mice of the present invention are for example those described in: Taylor, G. A., E. Carballo, et al. (1996) "A pathogenetic role for TNF alpha in the syndrome of cachexia, arthritis, and autoimmunity resulting from tristetraprolin (TTP) deficiency." Immunity 4(5): 445-454. In these mice the gene encoding TTP has been knocked out by inserting a neomycin resistance gene into the protein-coding region of the second exon of the gene encoding TTP, thereby introducing multiple stop codons upstream of the sequences encoding the two putative zinc fingers of TTP.

MRP14 single knockout mice which can be used to generate mice of the present invention are for example those described in: Manitz, M. P., B. Horst, et al. (2003). "Loss of MRP14 (S100A9) results in reduced interleukin-8-induced CD11b surface expression, a polarized microfilament system, and diminished responsiveness to chemoattractants in vitro." Mol Cell Biol 23(3): 1034-1043. In these mice the gene encoding MRP14 has been knocked out by replacing 760 bp of the genomic sequence containing exon 2 of the gene encoding MRP14 and the adjacent intron region with a pMC1-derived neomycin-poly(A) expression cassette, thereby deleting the initial start codon of the gene encoding MRP14.

The invention further relates to methods comprising the animal of the invention. In one embodiment the invention provides a method for identifying and/or testing a therapeutic agent for use in the prevention, treatment or alleviation of psoriasis, psoriatic arthritis, a psoriasis-like diseases and/or an inflammatory diseases of the bowel of a subject, wherein the method comprises:
(i) administering a test agent to the non-human double knockout animal of the invention; and
(ii) determining in a group of said non-human transgenic animals of the invention to which said test agent is administered at least one phenotype associated with said disease. A "group" consists of at least one animal. Preferably step (ii) further comprises determining at least one phenotype associated with said disease in a second group of the same non-human transgenic animals to which the test agent is not administered (control group). Also preferred is that said test agent is identified as therapeutic agent by comparing the results of step (ii) of the test group with the results of step (ii) of the control group. "Control group" preferably refers to animals to which a placebo is administered or nothing is administered. "Administering" refers to any mode of administration known to the person skilled in the art for therapeutically active agents. For example, "administering" refers to topical, dermal, transdermal, epicutaneous, peroral, enteral and parenteral. "Determining" refers to standard techniques known to the person skilled in the art, e.g. those described in the examples herein. "Subject" refers herein to an animal or human, wherein a human is preferred.

Moreover the invention relates to a method for testing a therapeutic agent for use in the prevention, treatment or alleviation of psoriasis, psoriatic arthritis, a psoriasis-like disease and/or inflammatory disease of the bowel of a subject, wherein the method comprises:
(i) administering a test agent under different administration conditions to the non-human transgenic animal of the invention;
(ii) determining for each administration condition at least one phenotype associated with said disease in a group of said non-human transgenic animals of the invention to which said test agent is administered;
(iii) identifying correlations between administrations conditions and the therapeutic effect of said agent.

"Different administration conditions" refer e.g. to different concentrations of the test agent, different administration times or administration intervals, or different pharmaceutical formulations comprising the test agent. "Phenotype associated with said disease" preferably refers the psoriatic phenotypes described herein.

The present invention further relates to therapeutic agents identified by a method comprising the non-human transgenic animal of the invention, e.g. the methods described above. Said therapeutic agent of the invention preferably alleviates at least one phenotype associated with psoriasis, psoriatic arthritis, psoriasis-like diseases and/or inflammatory diseases of the bowel. Accordingly the therapeutic agent of the invention is preferably for use in the prevention, treatment and/or alleviation of at least one disease of a subject, wherein said disease is psoriasis, psoriatic arthritis, a psoriasis-like disease and/or an inflammatory disease of the bowel. Said "subject" is preferably a human or animal. More preferably said subject is a human.

In addition the present invention further relates to a pharmaceutical composition comprising a therapeutic agent identified by a method comprising the non-human transgenic animal of the invention. In a preferred embodiment said pharmaceutical composition is for use in the prevention, treatment or alleviation of a disease of a subject, wherein said disease is psoriasis, psoriatic arthritis, a psoriasis-like disease and/or an inflammatory disease of the bowel. Also encompassed by the invention is a method for the treatment of a subject suffering from psoriasis, psoriasis, psoriatic arthritis, a psoriasis-like disease and/or an inflammatory disease of the bowel, wherein said method comprises administering a therapeutically effective amount of a therapeutic agent identified by a method comprising the non-human transgenic animal of the invention. Said "subject" is preferably a human or animal. More preferably said subject is a human. As used herein a "pharmaceutical composition" preferably contains at least one therapeutic agent in a therapeutically effective amount and at least one pharmaceutical excipient. By "therapeutically effective amount" is meant a dose that produces the effects for which the therapeutic agent is administered, preferably a prevention or alleviation of symptoms of psoriasis, psoriatic arthritis, psoriasis-like diseases and/or inflammatory diseases of the bowel.

The present invention further provides a method for the treatment of a subject suffering from psoriasis, psoriatic arthritis, a psoriasis-like disease and/or an inflammatory disease of the bowel, said method comprising administering a therapeutically effective amount of a therapeutic agent identified by a method comprising the animal of the invention.

In a further aspect the present invention relates to a tissue, isolated cell(s) or a cell line, characterized in that the cell(s) of said tissue, isolated cell(s) or cell line are deficient in the expression of the proteins TTP and MRP14. Said tissue, isolated cell(s) or a cell line is referred herein as "tissue, isolated cell(s) or a cell line of the invention".

"Tissue" as used herein refers to a tissue isolated from a non-human transgenic animal, preferably a non-human transgenic animal of the invention. "Isolated cell(s)" as used herein refers to a cell or cells isolated from a non-human transgenic animal, preferably a non-human transgenic animal of the invention. "Isolated" means separated from the non-human transgenic animal from which the tissue or cell is derived from. "Cell line" refers to an immortalized cell line from a multicellular organism, like an animal or human. Preferably said cell line is a cell line derived from a mammal. "Deficient in the expression of the proteins TTP and MRP14" means that tissue, isolated cell(s) or cell line of the invention do not express the proteins TTP and MRP14 at all or have a significantly reduced expression of the proteins TTP and MRP14 in compare to a control. "Significantly reduced expression in comparison to a control" means that cells of the tissue, isolated cell(s) or cell line of the invention express less than 50% in comparison to cells of a control (tissue, isolated cell(s) or cell line that is not modified in the expression of TTP and MRP14). Preferably the tissue, isolated cell(s) or cell line of the invention expresses less than 30% in comparison to cells of a control. More preferably the tissue, isolated cell(s) or cell line of the invention expresses less than 10% in comparison to cells of a control.

In one embodiment the tissue, isolated cell(s) or a cell line of the invention comprises a homozygous knockout of the gene encoding TTP and a homozygous knockout of the gene encoding MRP. The tissue, isolated cell(s) or cell line of the invention can be a tissue, isolated cell(s) or cell line derived from the non-human transgenic double knockout animal of the invention. Moreover the tissue, isolated cell(s) or a cell line can be obtained by (a) genetically modifying a non-transgenic animal, in that way that (i) the gene encoding TTP of said animal is knocked out on both TTP alleles, and (ii) the gene encoding MRP14 of said animal is knocked out both MRP14 alleles and (b) isolating a tissue and or cell(s) from the obtained double knockout animal. Moreover the tissue, isolated cell(s) or a cell line of the invention can be obtained by modifying a tissue or isolated cell(s) derived from a non-human transgenic animal that comprises no knockout of the gene encoding TTP and the gene encoding MRP14 or by modifying a cell line that comprises no knockout of the gene encoding TTP and the gene encoding MRP14. "Modifying" refers to any molecular biological method that leads to a decreased expression of TTP and MRP14, e.g. gene knockout of the genes encoding TTP and MRP14 or transient knockdown of TTP and MRP14. Also encompassed by the invention are methods comprising the inhibition of TTP and/or MRP14 by use of inhibitors which inhibit the transcription, translation or protein function of TTP and/or MRP14.

The present invention furthermore relates to uses of the non-human transgenic animal of the invention and the tissue, isolated cell(s) or cell line of the invention.

In one embodiment the present invention relates to the use of the non-human transgenic animal of the invention or the tissue, isolated cell(s) or cell line of the invention for identifying and/or characterizing molecular and cellular mechanisms underlying psoriasis, psoriatic arthritis, psoriasis-like diseases and/or inflammatory diseases of the bowel. Said diseases preferably refer to human diseases.

In another embodiment the present invention relates to the use of the non-human transgenic animal of the invention or the tissue, isolated cell(s) or cell line of the invention for use in evaluating the treatment of a disease selected from the group consisting of psoriasis, psoriatic arthritis, psoriasis-like diseases and/or inflammatory diseases of the bowel. Preferably said disease is a human disease.

In a further embodiment the present invention relates to the use of the non-human transgenic animal of the invention or the tissue, isolated cell(s) or cell line of the invention for use in identifying and/or testing a therapeutic agent or pharmaceutical composition for use in the prevention, treatment or alleviation of psoriasis, psoriatic arthritis, psoriasis-like diseases and/or an inflammatory diseases of the bowel. Said diseases preferably refer to human diseases.

The animal of the present invention is suitable for investigating the role of TNF in the development and/or severity of psoriasis, psoriatic arthritis, psoriasis-like diseases and/or inflammatory diseases of the bowel of a human or animal subject. For instance the animal of the present invention is suitable for investigating the correlation between systemically circulating TNF amounts and the severity of phenotypes associated with psoriasis, psoriatic arthritis, psoriasis-like diseases and/or inflammatory diseases of the bowel. Accordingly in one embodiment the present invention relates to the use of the non-human transgenic animal of the invention as an animal model for investigating the role of TNF in the pathology underlying psoriasis, psoriatic arthritis, psoriasis-like diseases and/or inflammatory diseases of the bowel.

Moreover the animal of the present invention is suitable for investigating the correlation between psoriasis, psoriatic arthritis and/or psoriasis-like diseases and inflammatory diseases of the bowel. Accordingly in one embodiment the present invention relates to the use of the non-human transgenic animal of the invention as an animal model for investigating the correlation between psoriasis, psoriatic arthritis and/or psoriasis-like diseases and inflammatory diseases of the bowel. Preferably the present invention provides an animal model for investigating the correlation between psoriasis and inflammatory diseases of the bowel.

The non-human transgenic animal of the invention represents an animal model for psoriasis, psoriatic arthritis, psoriasis-like diseases and/or inflammatory diseases of the bowel. Moreover the tissue, isolated cell(s) or cell line of the invention represent a cell model for psoriasis, psoriatic arthritis, psoriasis-like diseases and/or inflammatory diseases of the bowel. Accordingly, the present invention also relates to an animal model comprising the non-human transgenic animal of the invention and a cell model comprising the tissue, isolated cell(s) or cell line of the invention. Also encompassed by the invention is the use of the animal model of the invention and/or the cell model of the invention for the uses defined above for the animal, tissue, isolated cell(s) or cell line of the invention.

### EXAMPLES

The following examples are for illustrative purposes only and are not intended to limit the scope of the claims.

### Example 1

### - Materials and Methods -

### Generation and genotyping of mice

Animal studies were approved by the review board of the local Government. Mice were generated by crossing TTP-deficient₁₀ with MRP14-deficient₁₁ mice. All mice used were backcrossed into the C57BI6 background for at least 10 generations. Genotyping was performed by PCR using primers and conditions as described₁₀₋₁₁.

### Keratinocyte isolation

Primary epidermal keratinocytes were isolated from the skin of 2-3 days old newborn mice as described₂₂ and cultured in FAD medium containing 50µM calcium ions.

### Immunostaining

Sections (4µm) of paraffin-embedded skin were deparaffinized in xylene and rehydrated using standard procedures. Sections (5µm) of frozen tissue were fixed in ice-cold acetone. After blocking with 10% normal goat serum in phosphate-buffered saline (PBS) for 30 min, the sections were washed in PBS. Primary antibodies dissolved in PBS were applied for 1 hour at room temperature or overnight at 4°C. After washing in PBS, the sections were incubated with anti-rabbit or anti- rat Alexa 488-coupled IgG and propidium iodide diluted in PBS. Finally, after rinsing in phosphate-buffered saline, the sections were fixed with 4% paraformaldehyde and mounted in Mowiol. The following primary polyclonal antibodies were used: anti-keratin14 (1:100), anti-keratin 10 (1:100), anti-loricrin (1:2000) (Covance), Pan Keratin (c11) (1:100) (Cell Signaling)Rat monoclonal antibodies were used on frozen sections as follows: F4/80 (1:100; Acris), anti-Gr-1 (1:20; Ly- 6G and Ly-6c; BD Pharmingen), anti-CD3 (1:1000; eBioscience), Sections were counterstained with propidium iodide or DAPI to visualize the nuclei. Fluorescent stainings were analyzed using a Zeiss Axio Vision Microscope.

### Immunhistochemistry

Sections (4µm) of paraffin-embedded skin were stained with Rabbit antisera against recombinant murine MRP8 (produced as described previously₂₃) Immunohistochemistry was performed with an alkaline phosphatase detection kit using Vectastain ABC-AP, Vector Red Substance and secondary biotinylated antibodies (Vector Laboratories).

### Real-time quantitative PCR

RNA was extracted from snap-frozen mouse tissue using RNeasy columns and the TissueLyser II (Qiagen, Hilden, Germany) according to the manufacturer's instructions. cDNA was synthesized from 1 µg of total RNA using random hexanucleotide primers and the Revert Aid reverse transcription system (Thermo Scientific). Real-time quantitative PCR was performed in an ABI PRISM 7300 cycler (Life Technologies, Darmstadt, Germany) employing the murine beta-actin TaqMan gene expression assay (Life Technologies) as endogenous control as well as TaqMan gene expression assays specific for mouse IL-17A, S100A8, IL-23, IL-22, CD8, Foxp3, T-bet, GATA-3, Rorγt, GrzA, IL-23, IL-4, TGF-β, IFN-γ, TNF-α, S100A8, S100A9, TSLP, IL-10, IL-17, IL-22, IL-27, IL-21. All reported mRNA levels are normalized to beta-actin.

### Western Blot Analysis

To analyze MRP8 or p-ERK expression snap-frozen skin samples were homogenized using the Precellys Homogeniser (Peqlab). Homogenized samples were resuspended in NP40 buffer. Lysates were centrifuged at 14,000g for 15 minutes and supernatant was used for Western blot analysis. Equal amounts of protein were separated by SDS-PAGE and blotted onto Hybond-P polyvinylidine difluoride membranes (Amersham) with Rabbit antisera against recombinant murine MRP8 (produced as described previously23) or p-ERK Antibody (Cell Signaling). Protein bands were visualized with polyclonal goat anti-rabbit secondary antibody (Daco) on Hyperfilm (Amersham) using enhanced chemiluminescence (Amersham).

### Dot Blot

Supernatants of the skin from anti-TNF and IgG treated TTP_{-/-} x MRP14_{-/-} mice were coated onto Hybond-P polyvinylidine difluoride membranes(Amersham). After incubation with anti Mrp8 (produced as described previously₂₃) bands were visualized with polyclonal goat antirabbit secondary antibody (Daco) on Hyperfilm (Amersham) using enhanced chemiluminescence (Amersham).

### Anti-TNF treatment

To neutralize TNF in TTP_{-/-} x MRP14_{-/-} mice we used 40mg/kg Certolizumab (Cimzia; UCB) a PEGylated Fab' fragment of a humanized TNF-inhibitor monoclonal antibody already in clinical use for therapy of arthritis and psoriasis. We injected 30µl on day4, 6 and 8 into the back skin of newborn TTP_{-/-} x MRP14_{-/-} mice.

### Murine IL-17 measurment by Elisa

Mouse IL-17 were measured by Quantikine ELISA kit (R&D systems). Tissues were assayedwith the manufacturer's instructions.

### Assessment of the psoriasis like skin disease

To evaluate the severity of the psoriasis like phenotype of TTP^{-/-} x S100A9^{-/-} mice an adapted PASI (Psoriasis Area and Severity Index) score was used.
Grade 0: 0% involved area
Grade 1: <10% involved area
Grade 2: 10-29% involved area
Grade 3: 30-49% involved area
Grade 4: 50-69% involved area
Grade 5: 70-89% involved area
Grade 6: 90-100% involved area

### H&E staining

Skin or hind paw sampels were fixed in 4% paraformaldehyde. Fixed skin samples were embedded in paraffin according to procedures used for routine histology and stained with Hematoxilin and Eosin

### Toluidine Blue staining

Hind Paws of TTP-/- Mrp14-/- mice were fixed overnight in 4% formalin and then decalcified in 14% EDTA (Sigma-Aldrich). Paraffin sections of hind paws were embedded in paraffin according to procedures used for routine histology and stained with toluidine blue.

### Example 2

### - Detailed description of one way of carrying out the claimed invention -

The myeloid-related protein-14 (MRP14, MRP14) is a damage-associated molecular pattern (DAMP) molecule that together with its dimerization partner myeloid-related protein-8 (MRP8, S100A8) is increased in many inflammatory disorders and has been shown to have proinflammatory functions. So far, the expression of MRP8 and MRP14 have been regarded as being closely related, whilst the lack of MRP14 has been considered to also result in a functional knockout of MRP8. Here, we show that under inflammatory conditions, MRP8 and MRP14 have separable expression and functions, with MRP14 deficiency promoting tumor necrosis factor alpha (TNFalpha) dependent inflammation via unbalanced expression of MRP8. In TNFalpha overexpressing ttp-/- mice that show mild inflammation of the skin, the lack of MRP14 leads to a severe psoriasis-like skin disease with all major histological characteristics of human psoriasis. Psoriasis-like disease in these mice is accompanied by high levels of MRP8, mainly in keratinocytes, and leads to death within 2 weeks after birth. The expression of effector molecules known to be upregulated during the pathogenesis of psoriasis, including IL-17, IL-23 and IL-22, is markedly increased in ttp-/-/mrp14-/- as compared to TTP-/- mice. Psoriasis-like skin disease of ttp-/-/mrp14-/- mice is TNFalpha dependent as inhibition of TNFalpha using either specific antibodies or through the loss of TNFalpha in ttp-/-/mrp14-/-/tnf-/- mice, prevents the development of the psoriatic phenotype and early animal death. Moreover, in vitro analyses of murine keratinocytes demonstrate that increased expression of MRP8 in the absence of MRP14 is mediated by TNFalpha. Our results not only establish a novel model for human psoriasis but shed new light on the expression and function of the alarmins MRP8 and MRP14.

Psoriasis is a common disease that is characterised by complex interactions between immune cells and the epidermis₁. Currently, the pathogenesis of the disease is not fully understood. Recent evidence suggests that epidermal keratinocytes not only form a barrier against harmful organisms within the external environment, but also have functions in the disease onset and immune regulation of the skin. Recently the calcium-binding molecules MRP8 and MRP14 have come into focus within the pathogenesis of skin diseases such as dermatosis and psoriasis₂. In vivo, MRP8 and MRP14 form heterodimers and are mainly expressed in granulocytes and monocytes. Under inflammatory conditions, MRP8 and MRP14 are additionally expressed in mature macrophages and osteoclasts. MRP14 and its complex-partner MRP8 are expressed at very low levels in normal skin, however they become upregulated during psoriasis₃₋₅. Their expression can be induced by psoriasis relevant detergents, UV exposure and cytokines₆₋₇. Recently, MRP8 and MRP14 have been identified as factors which lead to the activation of hyperproliferative keratinocytes with disturbed differentiation₈₋₉.

To determine the extent of the involvement of these proteins in the development of psoriasis we crossed MRP14-deficient (mrp14_{-/-}) with Tristetraprolin deficient (ttp_{-/-}) mice. TTP-deficient mice were used as a systemic autoimmune inflammatory model, exhibiting an increased stability of TNFalpha mRNA and consequently higher levels of the cytokine at protein level. ttp_{-/-} mice appear normal at birth, but after months they develop marked medullary and extramedullary myeloid hyperplasia associated with cachexia, erosive arthritis, conjunctivitis and dermatitis₁₀. Mrp14 deficient mice are healthy and develop normally. Mating of ttp_{-/-} with mrp14_{-/-} mice resulted in ttp_{-/-}/mrp14_{-/-} pups that also appeared normal at birth and showed no differences compared to wild type or control littermates until postnatal day 4-5. At around day 5, ttp_{-/-}/mrp14_{-/-} mice started to develop the first signs of skin abnormalities, which to our surprise were far more severe that those seen in ttp_{-/-} mice after months. By day 8-9 after birth, ttp_{-/-} /mrp14_{-/-} animals had developed hard, inflamed, inflexible skin with widespread scaling that all together presented as psoriasis-like changes (Fig.1 a). The adapted Psoriasis Area and Severity Index (PASI) illustrates the rapid disease progression of ttp_{-/-}/mrp14_{-/-} mice within a few days from PASI-Index 0 to the highest PASI-Index 6 (Fig.1b). The comparison of the different genotypes at day 9 demonstrated that macroscopically, 70% of newborn ttp_{-/-} mice have a normal phenotype and show no evidence of disease. About 30% of ttp_{-/-} mice exhibit a very slight scaling and at histological analysis show a slight thickening of the epidermis accompanied by a low elevation of macrophages and granulocytes within the dermis (Fig.1c), which return to the basal levels found in control littermates after 2-3 days. In contrast, the loss of mrp14_{-/-} in ttp_{-/-} mice resulted in a strong psoriatic-like phenotype. Histological and immunohistological staining of skin sections from ttp_{-/-}/mrp14_{-/-} mice demonstrates all characteristics common to human psoriasis (Fig.1c). Compared to ttp_{-/-} mice, ttp_{-/-}/mrp14_{-/-} mice showed a markedly thickened epidermis (acanthosis), with loss of the granular layer, thickening of the cornified layer (hyperkeratosis) and parakeratosis as a result of incomplete keratinocyte differentiation (H/E staining Fig.1c). Keratin 14 (K14), which is normally expressed in the basal layer, was suprabasally expressed in all layers of the skin of ttp_{-/-}/mrp14_{-/-} mice, which is a hallmark of an inflamed, hyperproliferative epidermis. In contrast, expression of keratin 10 (K10) and the terminal differentiation marker loricrin (Lor) was markedly reduced, thus indicating incomplete keratinocyte-differentiation. H/E staining of ttp_{-/-}/mrp14_{-/-} mice revealed an increased cellularity in the dermis, which is highly dependant on immune cell invasion. As typical for psoriasis, immunohistological analysis revealed an accumulation of macrophages, granulocytes and CD3-positive T cells as well as the presence of invading granulocytes under the stratum corneum within the epidermis (Munroe microabscesses).

It has been demonstrated in several studies that MRP8 and MRP14 function as damage associated molecular pattern (DAMP) molecules, with both proteins strongly interacting to form a pro-inflammatory complex. Moreover, under physiological conditions the absence of MRP14 has been shown to result in the loss of its binding partner MRP8, which is why mrp14₋₁₋ mice have been postulated to constitute functional double knockout for MRP8 and MRP14₁₁₋₁₂. Based on these data, we next examined the expression of MRP8 in the skin of our mice by immunohistochemistry. Unexpectedly, we found a high accumulation of MRP8 in the hyperproliferative epidermis of ttp_{-/-}/mrp14_{-/-} mice compared to the skin of mrp14_{-/-} and wild type mice (Fig.2a). Furthermore, Real-time quantitative PCR analysis (Fig.2b) revealed a significant increase in MRP8 expression in the skin of ttp_{-/-}/mrp14_{-/-} mice, suggesting that under chronic inflammatory conditions of ttp_{-/-} mice, MRP14 deficiency leads to a strong expression of its complex-partner MRP8 within the skin. As numerous studies have demonstrated that the cytokines IL-17, IL-22 and IL-23 play an important role in the pathogenesis of psoriasis₁₃₋₁₅, we next measured the mRNA-expression of these cytokines in skin of our different genotypes using real time RT-PCR (Fig.2c). We found expression of these cytokines in the skin of ttp_{-/-} mice, but the levels appeared not to be sufficient to cause psoriasis-like disease. In contrast, the loss of Mrp14 in ttp_{-/-}/mrp14_{-/-} mice provoked a clear increase in cytokine expression in comparison to the levels observed in ttp_{-/-} mice. IL-17, which has been widely considered as a major cytokine in psoriasis,₁₆₋₁₉ was most strongly increased in the skin of ttp_{-/-}/mrp14_{-/-} mice compared to that of ttp_{-/-} mice. Our IL-17 mRNA expression data were confirmed at protein level using ELISA, again showing a significantly elevated expression of IL-17 in ttp_{-/-}/mrp14_{-/-} mice (35,6pg/ml) as compared to ttp_{-/-} (5,4pg/ml), mrp14^{-/-}(2,1pg/ml) and WT (2,4pg/ml) mice (Fig.2d).

These findings lead to the question of whether chronic overexpression of TNFalpha, which has been considered a key cytokine in psoriasis₂₀₋₂₁ , may be responsible for the psoriasislike phenotype of the ttp_{-/-}/mrp14_{-/-} mice. To this end, we treated ttp_{-/-}/mrp14_{-/-} mice with neutralizing antibodies against TNFalpha that have been successfully applied in the treatment of human disease. Of note, treatment of ttp_{-/-}/mrp14₋₁₋ with anti-TNFalpha antobodies mice led to a significant improvement of their psoriasis-like phenotype when compared to control IgG treated ttp_{-/-}/mrp14_{-/-} mice. Macroscopically, the treatment greatly suppressed the thickening and scaling of the skin (Fig.3a top panel), which is displayed quantitatively using the adapted PASI score (Fig.3b). This was confirmed in histological analyses, where the signs of psoriasis-like skin disease in ttp_{-/-}/mrp14_{-/-} mice (Fig.3a left panel) were greatly reduced compared to those of IgG treated mice (Fig.3a right panel). The skin was thinner, and its structure appeared very close to normal. K14 expression was restricted to the basal layer and K10 and Lor were present only in the epidermis. Invasion of macrophages, granulocytes and CD3-positive T-cells into the dermis was also diminished. The expression of the MRP14 complex-partner MRP8 was reduced in the epidermis after Anti-TNF treatment (Fig.3a), and was confirmed by low MRP8 protein levels in the skin of Anti-TNF treated ttp_{-/-}/mrp14_{-/-} mice (Fig.3c). Quantitative PCR analysis demonstrated a reduced expression of the psoriasis relevant cytokines IL-17, IL23, IL-22 (Fig.3d-f) at mRNA level.. At protein level, the anti-TNF treated ttp_{-/-}/mrp14_{-/-} mice showed a significantly lower skin expression of IL-17 compared to the skin of IgG controls (Fig.3g).

As TTP has been demonstrated to regulate the mRNA stability of different cytokines distinct from TNFalpha, we next sought confirmation that the loss of TNFalpha would prevent the development of a psoriasis-like phenotype in ttp_{-/-}/mrp14_{-/-} mice. To this end we generated ttp_{-/-}/mrp14_{-/-}/tnfalpha_{-/-} triple knockout mice by crossing ttp_{-/-}/mrp14_{-/-} mice with tnfalpha deficient animals. Confirming our antibody data, we found through macroscopical analysis, that TNF deficiency led to a complete suppression of the psoriatic phenotype in ttp_{-/-}/mrp14_{-/-}mice with ttp_{-/-}/mrp14_{-/-}/tnfalpha_{-/-}mice showing no obvious differences to wild type animals (Fig.4a top panel). Histological analysis of the skin demonstrated normal skin development and expression of MRP8, K14, K10 and Lor, normal keratinocyte differentiation and proliferation of the epidermis. Also, no invading macrophages (F4/80), granulocytes (Gr1) and CD3-positive T cells were found in the dermis of in ttp_{-/-}/mrp14_{-/-}/tnfalpha_{-/-} mice. To finally test the hypothesis that TNFalpha exerts direct effects on the accumulation of Mrp8 in the skin of ttp_{-/-}/mrp14_{-/-} mice, we isolated keratinocytes from wild type and Mrp14_{-/-} mice and treated these cells with TNFalpha (Fig.4b). Western Blot analysis demonstrated that TNFalpha induces only a weak expression of Mrp8 in wild type keratinocytes but a strong and considerably higher expression in mrp14_{-/-} keratinocytes.

These findings show that the psoriasis-like phenotype in ttp_{-/-}/mrp14_{-/-} mice is triggered through TNFalpha-dependent inflammation. The loss of TNFalpha activity, either via administration of TNFalpha-neutralizing antibody or through using TNFalpha-deficient mice supresses the development and protects mice from the development of psoriasis-like skin disease. Morover, the outbreak of the psoriasis-like disease depends on the loss of MRP14. Overall our results indicate that MRP14 is required for skin homeostasis and has an essential regulatory function during skin inflammation. MRP8 and MRP14 represent an important new aspect in the pathogenesis of psoriasis and a novel target in disease treatment.

The invention may be practiced otherwise than as particularly described in the foregoing description and examples. Numerous modifications and variations of the present invention are possible in light of the above teachings and, therefore, are within the scope of the appended claims.

### REFERENCES

### References of the description:

Binus A. M., Han J., Qamar A. A., Mody E. A., Holt E. W., Qureshi A. A. (2011). Associated comorbidities in psoriasis and inflammatory disease of the bowel. J. Eur. Acad. Dermatol. Venereol. 26(5):644-50.
Broome, A. M., D. Ryan, et al. (2003). "S100 protein subcellular localization during epidermal differentiation and psoriasis." J Histochem Cytochem 51(5): 675-685.
Cho J. H., Brant S. R. (2011). Recent insights into the genetics of inflammatory disease of the bowel. Gastroenterology 140, 1704-1712.
Danese S., Semeraro S., Papa A., Roberto I., Scaldaferri F., Fedeli G., Gasbarrini G., Gasbarrini A. (2005). Extraintestinal manifestations in inflammatory disease of the bowel. World J. Gastroenterol. 11, 7227-7236.
Georgiou G., Pasmatzi E., Monastirli A., Tsambaos D. (2006). Cutaneous manifestations of inflammatory disease of the bowel. Hosp. Chron. 1, 158-168.
Hall B, Limaye A, Kulkarni AB (2009) Overview: generation of gene knockout mice. Curr Protoc Cell Biol. Chapter 19
Huang BL, Chandra S, Shih DQ. (2012) Skin manifestations of inflammatory disease of the bowel. Front Physiol. 2012 Feb 6;3:13.
Humbert P, Bidet A, Treffel P, Drobacheff C, Agache P (July 1991). "Intestinal permeability in patients with psoriasis". J. Dermatol. Sci. 2 (4): 324-6.
Manitz, M. P., B. Horst, et al. (2003). "Loss of MRP14 (MRP14) results in reduced interleukin-8-induced CD11 b surface expression, a polarized microfilament system, and diminished responsiveness to chemoattractants in vitro." Mol Cell Biol 23(3): 1034-1043.
Maston GA, Evans SK, Green MR (2006) Transcriptional regulatory elements in the human genome. Annu Rev Genomics Hum Genet. 7:29-59.
Mirmohammadsadegh, A., E. Tschakarjan, et al. (2000). "Calgranulin C is overexpressed in lesional psoriasis." J Invest Dermatol 114(6): 1207-1208.
Peslyak MY, Model of pathogenesis of psoriasis. Part 1. Systemic psoriatic process, 2009-2012, p. 20, ISBN 5905504024, 9785905504020
Quekenborn-Trinquet, V., P. Fogel, et al. (2005). "Gene expression profiles in psoriasis: analysis of impact of body site location and clinical severity." Br J Dermatol 152(3): 489-504
Skroza N, Proietti I, Pampena R, La Viola G, Bernardini N, Nicolucci F, Tolino E, Zuber S, Soccodato V, Potenza C (2013). "Correlations between psoriasis and inflammatory diseases of the bowel " Biomed Res Int.; 2013:983902.
Taylor, G. A., E. Carballo, et al. (1996). "A pathogenetic role for TNF alpha in the syndrome of cachexia, arthritis, and autoimmunity resulting from tristetraprolin (TTP) deficiency." Immunity 4(5): 445-454.
Timani S., Mutasim D. F. (2008). Skin manifestations of inflammatory disease of the bowel. Clin. Dermatol. 26, 265-273.

### References of the examples:

1. Nickoloff, B. J. and F. O. Nestle (2004). "Recent insights into the immunopathogenesis of psoriasis provide new therapeutic opportunities." J Clin Invest 113(12). 1664-1675.
2. Foell, D., H. Wittkowski, et al. (2007). "S100 proteins expressed in phagocytes: a novel group of damage-associated molecular pattern molecules." J Leukoc Biol 81(1), 28-37.
3. Broome, A. M., D. Ryan, et al. (2003). "S100 protein subcellular localization during epidermal differentiation and psoriasis." J Histochem Cytochem 51(5): 675-685.
4. Mirmohammadsadegh. A., E. Tschakarjan, et al. (2000). "Calgranulin C is overexpressed in lesional psoriasis" J Invest Dermatol 114(6): 1207-1208.
5. Quekenbom-Trinquet, V., P. Fogel, et al. (2005). "Gene expression profiles in psoriasis: analysis of impact of body site location and clinical severity." Br J Dermatol 152(3): 489-504.
6. Boniface, K., J. C. Lecron, et al. (2005). "Keratinocytes as targets for interleukin-10-related cytokines: a putative role in the pathogenesis of psoriasis." Eur Cytokine Netw 16(4): 309-319.
7. Mationnet, C., F. Bemerd, et al. (2003). "Modulation of gene expression induced in human epidermis by environmental stress in vivo." J Invest Dermatol 121(6), 1447-1458.
8. Zenz, R., R Eferl, et al. (2005). "Psoriasis-like skin disease and arthritis caused by inducible epidermal deletion of Jun proteins." Nature 437(7057): 369-375.
9. Benoit, S., A. Toksoy, et al. (2006). "Elevated serum levels of calcium-binding S10 proteins A8 and A9 reflect disease activity and abnormal differentiation of keratinocytes in psoriasis." Br J Dermatol 155(1): 62-66.
10. Taylor, G. A., E. Carballo, et al. (1996). "A pathogenetic role for TNF alpha in the syndrome of cachexia, arthritis, and autoimmunity resulting from tristetraprolin (TTP) deficiency." Immunity 4(5): 445-454.
11. Manitz, M. P., B. Horst, et at. (2003). "Loss of S100A9 (MRP14) results in reduced interteukin-8-induced CD11b surface expression, a polarized microfilament system, and diminished responsiveness to chemoattractants in vitro." Mol Cell Biol 23(3); 1034-1043.
12. Vogl, T., K. Tenbrock, et al. (2007). "Mrp8 and Mrp14 are endogenous activators of Toll-like receptor 4, promoting lethal, endotoxin-induced shock." Nat Med 13(9): 1042-1049.
13. Blauvelt, A (2008). "T-helper 17 cells in psoriatic plaques and additional genetic links between IL-23 and psoriasis." J Invest Dermatol 128(5): 1064-1067.
14. Di Cesare, A., P. Di Meglio, et al. (2009). "The IL-23/Th17 axis in the immunopathogenesis of psoriasis." J Invest Dermatol 129(6), 1339-1350.
15. Harper, E. G., C. Guo, et al. (2009). "Th17 cytokines stimulate CCL20 expression in keratinocytes in vitro and in vivo: implications for psoriasis pathogenesis" J Invest Dermatol 129(9): 2175-2183.
16. Wilson, N. J., K. Boniface, et al. (2007). "Development, cytokine profile and function of human interleukin 17-producing helper T cells." Nat Immunol 8(9) 950-957.
17. Zheng, Y., D. M. Danilenko, et al. (2007). "Interleukin-22, a T(H)17 cytokine, mediates IL-23-induced dermal inflammation and acanthosis." Nature 445(7128): 648-651.
18. El Mali, K., S. H. Karbach, et al. (2012). "An Alternative Pathway of Imiquimod-Induced Psoriasis-Like Skin Inflammation in the Absence of Interleukin-17 Receptor A Signaling." J Invest Dermatol.
19. Johnston, A., Y. Fritz, et al. (2013). "Keratinocyte overexpression of IL-17C promotes psoriasiform skin inflammation." J Immunol 190(5): 2252-2262.
20. Pasparakis, M., G. Courtois, et al. (2002). "TNF-mediated inflammatory skin disease in mice with epidermis-specific deletion of IKK2." Nature 417(6891); 861-866.
21. Stratis, A, M. Pasparakis, et al. (2006) "Pathogenic role for skin macrophages in a mouse model of keratinocyte-induced psoriasis-like skin inflammation." J Clin Invest 116(8): 2094-2104.
22. Rope, E., Weinberg, W., Watt, F. M. & Land, H. p19ARF-independent induction of p53 and cell cycle arrest by Raf in murine keratinocytes. EMBO Rep.2, 145-150 (2001).
23. Goebeler M, Roth J, Burwinkel F, Vollmer E, Bocker W, Sorg C. Expression and complex formation of S100-like proteins MRP8 and MRP14 by macrophages during renal allograft rejection. Transplantation 1994; 58 :355-61

## Claims

1. A non-human transgenic double knockout animal, in which the following genes are knocked out
(i) gene encoding Tristetraprolin (TTP); and
(ii) gene encoding myeloid-related protein-14 (Mrp14; S100A9).

2. The non-human transgenic animal of claim 1, wherein
(a) the knockout of the gene encoding TTP is a homozygous knockout and the knockout of the gene encoding MRP14 is a homozygous knockout;
(b) the knockout of the gene encoding TTP is a homozygous knockout and the knockout of the gene encoding MRP14 is a heterozygous knockout;
(c) the knockout of the gene encoding TTP is a heterozygous knockout and the knockout of the gene encoding MRP14 is a homozygous knockout; or
(d) the knockout of the gene encoding TTP is a heterozygous knockout and the knockout of the gene encoding MRP14 is a heterozygous knockout.

3. The non-human transgenic animal of claim 1 to 2, exhibiting at least one phenotype associated with a disease selected from the group consisting of psoriasis, psoriatic arthritis, psoriasis-like diseases and/or an inflammatory disease of the bowel, wherein said disease is preferably a human disease.

4. The non-human transgenic animal of claim 3, wherein the inflammatory disease of the bowel is selected from the group consisting of Colitis ulcerosa, Crohn's disease, Collagenous colitis, Lymphocytic colitis, Ischaemic colitis, Diversion colitis, Behçet's disease, and colitis indeterminata.

5. The non-human transgenic animal of claims 3 to 4, wherein said animal develops a macroscopically observable expression of said phenotype postnatally, or wherein said phenotype is prevented and/or alleviated by suppressing or inhibiting Tumor necrosis factor alpha (TNF) in said animal, wherein TNF is preferably inhibited by subjecting an anti-TNF therapy to said animal, wherein said anti-TNF therapy preferably comprises administering a TNF neutralizing antibody to said animal.

6. The non-human transgenic animal according to any one of claims 1 to 5, wherein said animal is a non-human mammal or a fish.

7. The non-human transgenic animal according to claim 6, wherein said animal is a mouse, rat, rabbit, guinea pig, or zebrafish.

8. The non-human transgenic animal according to any of claims 1 to 7, for use in identifying and/or testing compounds for use in the prevention, treatment or alleviation of a disease selected from the group consisting of psoriasis, psoriatic arthritis, psoriasis-like diseases and inflammatory diseases of the bowel.

9. A method for generating a non-human transgenic double knockout animal according to any of claims 1 to 8.

10. The method of claim 9, comprising:
(i) crossing a TTP single knockout animal with a MRP14 single knockout animal of the same species; and optionally
(ii) backcrossing of the descendants obtained in step (i) into a genetic background strain.

11. A method for identifying and/or testing a therapeutic agent for use in the prevention, treatment or alleviation of psoriasis, psoriatic arthritis, a psoriasis-like disease and/or an inflammatory diseases of the bowel of a subject, the method comprising:
(i) administering a test agent to the non-human transgenic animal according to any one of claims 1 to 8; and
(ii) determining in a group of said non-human double knockout animals to which the test agent is administered at least one phenotype associated with psoriasis, psoriatic arthritis, psoriasis-like diseases and inflammatory diseases of the bowel, wherein said disease is preferably a human disease.

12. A tissue, isolated cell(s) or a cell line, **characterized in that** the cell(s) of said tissue, isolated cell(s) or cell line are deficient in the expression of the proteins TTP and MRP14.

13. A tissue, isolated cell(s) or cell line derived from the non-human transgenic animal according to any one of claims 1 to 8.

14. Use of the non-human transgenic animal according any one of claims 1 to 8 or the tissue, isolated cell(s) or cell line of claim 12 or 13 for identifying and/or characterizing molecular and cellular mechanisms underlying psoriasis, psoriatic arthritis, psoriasis-like diseases and/or inflammatory diseases of the bowel, wherein said disease is preferably a human disease.

15. Use of the non-human transgenic animal according any one of claims 1 to 8 or the tissue, isolated cell(s) or cell line of claim 12 or 13 for identifying and/or testing a therapeutic agent or pharmaceutical composition for use in the prevention, treatment or alleviation of psoriasis, psoriatic arthritis, psoriasis-like diseases and/or an inflammatory diseases of the bowel, wherein said disease is preferably a human disease.
